# EUROPEAN PATENT APPLICATION

(11) **EP 4 464 691 A2**
(43) Date of publication of application: **20.11.2024**
(21) Application number: 23715409.1
(22) Date of filing: 13.01.2023
(51) Int. Cl.: C07C 319/22, C07C 321/28, C08F 12/04, C08F 12/30, C08F 12/32, C08F 38/00, G02C 7/00, C08G 75/045, G02B 1/04

(54) **NAPHTHALENE COMPOUND, SYNTHESIS METHOD THEREFOR, AND COMPOSITION CONTAINING SAID NAPHTHALENE COMPOUND**

(30) Priority: 14.01.2022 JP 2022004119
(71) Applicant: Shikoku Chemicals Corporation, Marugame-shi, Kagawa 763-8504 (JP)
(72) Inventor: KUMANO, Takeshi, Ayauta-gun, Kagawa 769-0202 (JP); OGAWA, Aya, Ayauta-gun, Kagawa 769-0202 (JP); SHIOIRI, Ryosuke, Ayauta-gun, Kagawa 769-0202 (JP); KASHIWABARA, Takashi, Ayauta-gun, Kagawa 769-0202 (JP); AOKI, Kazunori, Ayauta-gun, Kagawa 769-0202 (JP)
(74) Representative: Müller-Boré & Partner Patentanwälte PartG mbB
(86) International application number: PCT/JP2023/000701
(87) International publication number: WO 2023/058781

(57) **Abstract**

This invention provides a novel naphthalene compound, a method for synthesizing the naphthalene compound, and a composition containing the naphthalene compound. The composition of the present invention can be applied to coating materials, ink, adhesives, tackifiers, gas barrier films, color filters, optical films, optical lenses, and touch panels. The invention relates to a naphthalene compound represented by chemical formula (I), a method for synthesizing the naphthalene compound, and a composition containing the naphthalene compound: 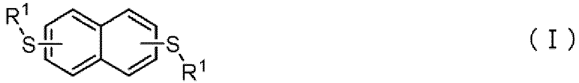 wherein R¹s (i) are the same or different and are each a group represented by formula (1), (ii) are the same and are each a group represented by formula (2), or (iii) are the same and are each a group represented by formula (3): 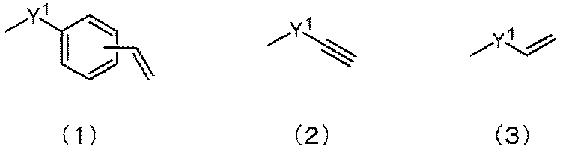 wherein Y¹s are the same and are each a single bond or a C₁-C₁₀ alkylene group.

## Description

### Technical Field

The present invention relates to a novel naphthalene compound, a method for synthesizing the naphthalene compound, and a composition containing the naphthalene compound.

### Background Art

A variety of materials have conventionally been used in resins for optical materials for use in various optical films, optical lenses, etc. In general, such materials are polymers of polymerizable monomers, whose polymerization proceeds with radical species; materials with a high refractive index, such as those containing a polymerizable functional group (e.g., sulfur-containing compounds and fluorene compounds), are suitable for improving light extraction efficiency (e.g., PTL 1 and PTL 2). However, the resins for optical materials reported so far still have room for improvement in terms of, for example, low thermal expansion properties, heat resistance, and mechanical strength of their cured products.

Further, no examples have been known so far in which a high-refractive-index naphthalene compound having both a naphthalene skeleton and a sulfur element in the molecule is used as a resin for optical materials; additionally, analogues with two polymerizable functional groups introduced in the molecule have not been reported.

### Citation List

### Patent Literature

PTL 1: JP2008-94987A
PTL 2: JP2010-248358A

### Summary of Invention

### Technical Problem

An object of the present invention is to provide a novel naphthalene compound, a method for synthesizing the naphthalene compound, and a composition containing the naphthalene compound.

### Solution to Problem

The present inventors conducted extensive research to achieve the object and recognized that a naphthalene compound obtained by reacting a specific naphthalene dithiol compound with a specific styrene compound, alkyne compound, or alkene compound can achieve the intended purpose. The inventors then completed the present invention.

Specifically, the first invention is a naphthalene compound represented by chemical formula (I): 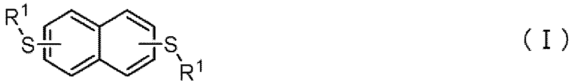 wherein R¹s (i) are the same or different and are each a group represented by formula (1), (ii) are the same and are each a group represented by formula (2), or (iii) are the same and are each a group represented by formula (3): 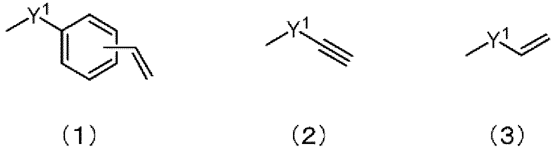 wherein Y¹s are the same and are each a single bond or a C₁-C₁₀ alkylene group.

The second invention is a method for synthesizing the naphthalene compound of the first invention,
the method comprising reacting a naphthalene dithiol compound represented by chemical formula (II) with a styrene compound represented by chemical formula (III), an alkyne compound represented by chemical formula (IV), or an alkene compound represented by chemical formula (V): 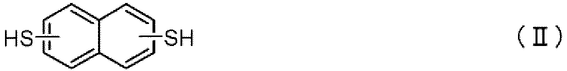 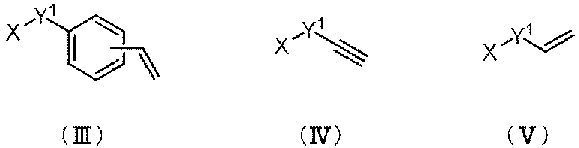 wherein Y¹ is as defined above, and X is a halogen atom (e.g., a fluorine atom, a chlorine atom, a bromine atom, or an iodine atom).

The third invention is a method for synthesizing the naphthalene compound of the first invention, the naphthalene compound being represented by chemical formula (I) wherein R¹s are the same and are each a group represented by formula (3),
the method comprising reacting a naphthalene dithiol compound represented by chemical formula (II) with a C₂-C₁₂ dihaloalkane compound to haloalkylate two thiol groups (first step), and reacting the resulting compound with a base (second step).

The fourth invention is a composition containing the naphthalene compound of the first invention.

### Advantageous Effects of Invention

The naphthalene compound of the present invention is a novel compound and is characterized by having a high refractive index. The naphthalene compound has two polymerizable groups in the molecule and is thus useful as a material for optical applications. The naphthalene compound of the present invention is also expected to be more compatible with various solvents and resin components than conventional materials for optical applications.

A composition containing the naphthalene compound of the present invention is expected to provide a cured product with lower thermal expansion properties, higher thermal resistance, and better mechanical strength, compared with conventional compositions, and is also expected to provide a cured product with a higher refractive index.

The composition of the present invention can be applied to coating materials, ink, adhesives, tackifiers, gas barrier films, color filters, optical films, optical lenses, touch panels, and the like.

### Brief Description of Drawings

Fig. 1: An IR spectrum chart of the white crystals obtained in Example 1.
Fig. 2: An IR spectrum chart of the pale yellow crystals obtained in Example 2.
Fig. 3: An IR spectrum chart of the pale yellow solid obtained in Example 3.
Fig. 4: An IR spectrum chart of the white crystals obtained in Example 4.
Fig. 5: An IR spectrum chart of the pale yellow crystals obtained in Example 5.
Fig. 6: An IR spectrum chart of the pale yellow liquid obtained in Example 6.
Fig. 7: An IR spectrum chart of the pale yellow crystals obtained in Example 7.
Fig. 8: An IR spectrum chart of the pale yellow liquid obtained in Example 8.
Fig. 9: An IR spectrum chart of the white crystals obtained in Example 9.

### Description of Embodiments

The present invention is described in detail below.

### 1. Naphthalene Compound

The present invention relates to the naphthalene compound represented by chemical formula (I) described above (which may be referred to below as "the naphthalene compound of the present invention").

The naphthalene compound represented by chemical formula (I) encompasses naphthalene compounds represented by chemical formula (1-1) to chemical formula (I-5). 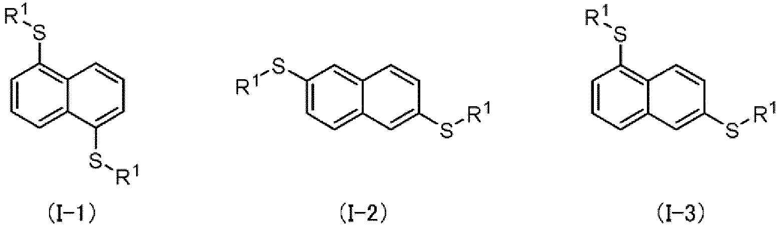 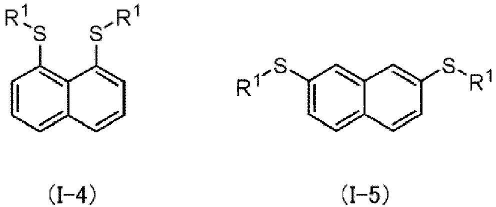 In the formulas, R¹s are as defined above.

Examples of the naphthalene compound represented by chemical formula (1-1) include naphthalene compounds represented by chemical formula (I-1-1) to chemical formula (1-1-9).

Examples of the naphthalene compound represented by chemical formula (1-2) include naphthalene compounds represented by chemical formula (I-2-1) to chemical formula (I-2-9).

Examples of the naphthalene compound represented by chemical formula (I-3) include naphthalene compounds represented by chemical formula (I-3-1) to chemical formula (I-3-12).

Examples of the naphthalene compound represented by chemical formula (I-4) include naphthalene compounds represented by chemical formula (I-4-1) to chemical formula (I-4-9).

Examples of the naphthalene compound represented by chemical formula (I-5) include naphthalene compounds represented by chemical formula (I-5-1) to chemical formula (I-5-9).

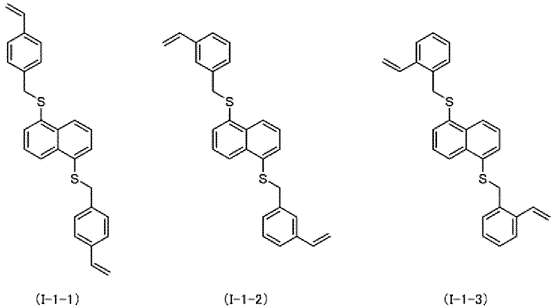

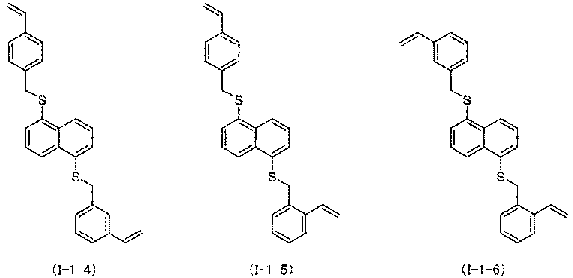

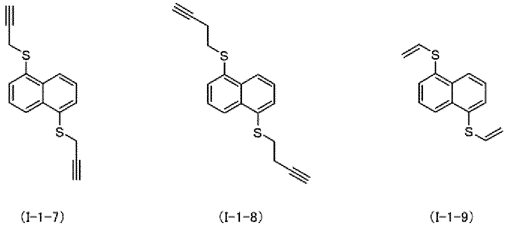

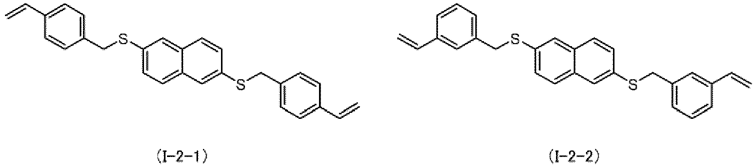

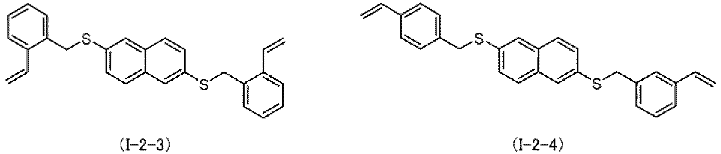

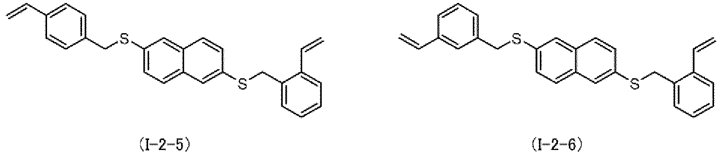

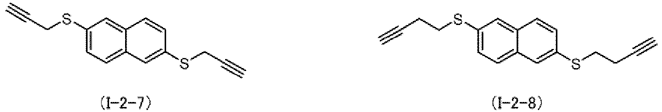

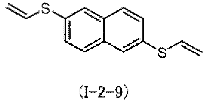

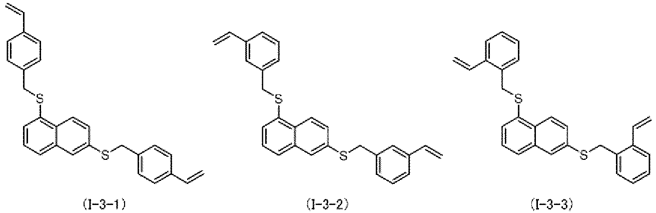

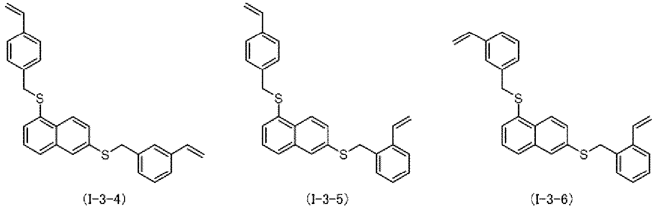

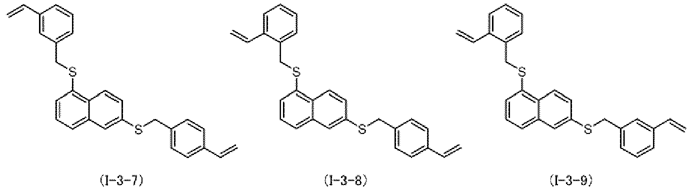

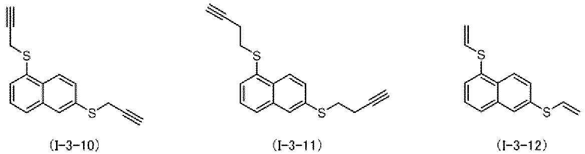

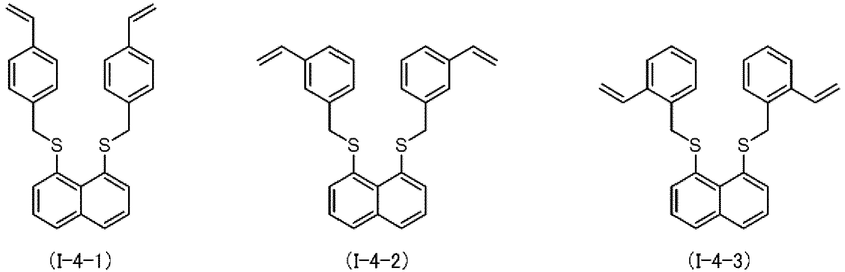

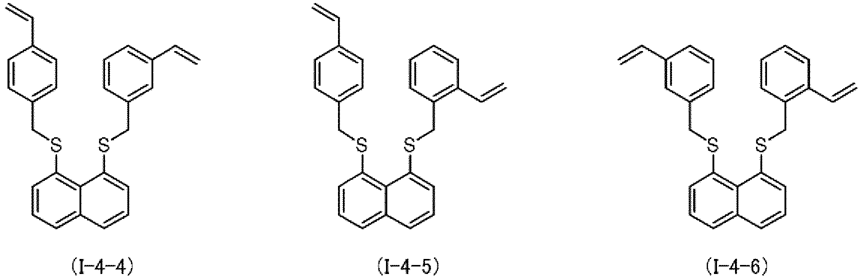

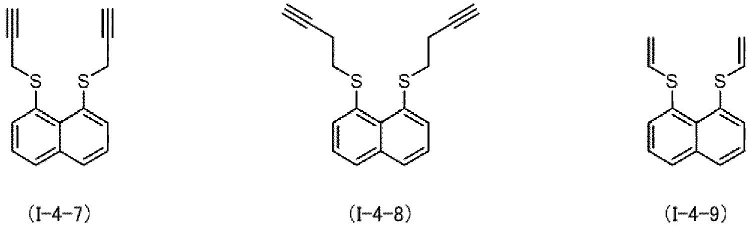

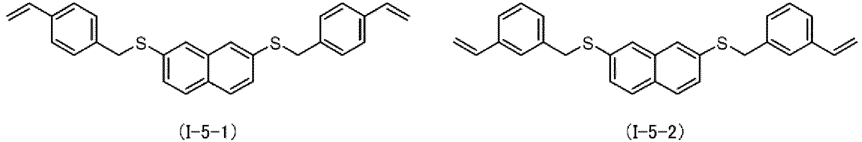

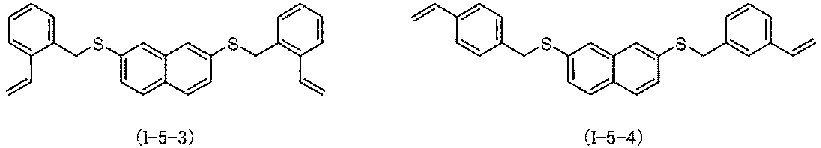

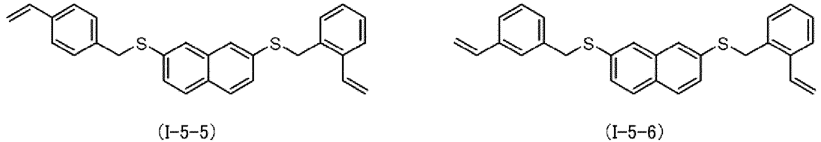

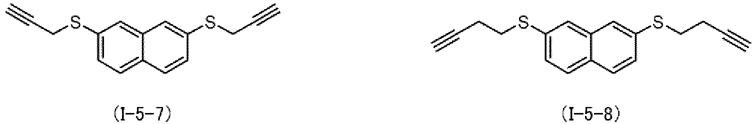

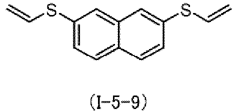

The group represented by formula (1) represented by R¹ includes groups represented by formulas (1-1) to (1-3). 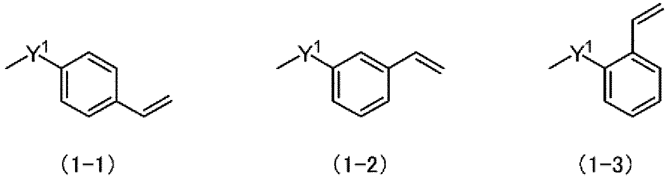 In the formulas, Y¹ is as defined above.

In the naphthalene compound of the present invention, it is preferred that R¹s (i) are the same or different and are each a group represented by any one of formulas (1-1) to (1-3), (ii) are the same and are each a group represented by formula (2), or (iii) are the same and are each a group represented by formula (3).

The C₁₋₁₀ alkylene group represented by Y¹ includes linear or branched C₁₋₁₀ alkylene groups. Specific examples include a methylene group, a methyl methylene group, a dimethylene group, a trimethylene group, an ethyl methylene group, a dimethyl methylene group, a tetramethylene group, a pentamethylene group, a hexamethylene group, a heptamethylene group, an octamethylene group, a nonamethylene group, and a decamethylene group. In the naphthalene compound of the present invention, it is preferred that Y¹s are the same and are each a C₁-C₆ alkylene group, more preferably a C₁-C₄ alkylene group, and particularly preferably a C₁-C₃ alkylene group.

Examples of synthesis methods for the naphthalene compound of the present invention include synthesis methods (1) and (2).

### Synthesis Method (1)

The naphthalene compound of the present invention can be synthesized by reacting a naphthalene dithiol compound represented by chemical formula (II) with a styrene compound represented by chemical formula (III), an alkyne compound represented by chemical formula (IV), or an alkene compound represented by chemical formula (V) (see reaction scheme (A) described below as a specific example).

The naphthalene dithiol compound represented by chemical formula (II) encompasses naphthalene dithiol compounds represented by chemical formula (II-1) to chemical formula (II-5) .

The naphthalene dithiol compound represented by chemical formula (II-1) is a precursor of the naphthalene compound represented by chemical formula (I-1), the naphthalene dithiol compound represented by chemical formula (II-2) is a precursor of the naphthalene compound represented by chemical formula (I-2), the naphthalene dithiol compound represented by chemical formula (II-3) is a precursor of the naphthalene compound represented by chemical formula (I-3), the naphthalene dithiol compound represented by chemical formula (II-4) is a precursor of the naphthalene compound represented by chemical formula (I-4), and the naphthalene dithiol compound represented by chemical formula (II-5) is a precursor of the naphthalene compound represented by chemical formula (I-5).

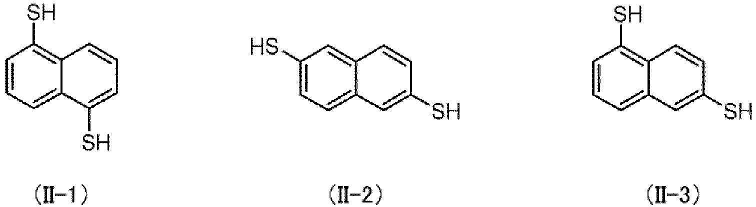

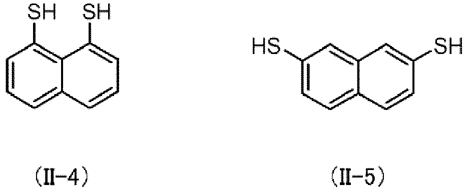

These naphthalene dithiol compounds for use may be commercially available reagents.

Examples of the halogen atom represented by X in the styrene compound represented by chemical formula (III) include a fluorine atom, a chlorine atom, a bromine atom, and an iodine atom. Preferred is a chlorine atom, a bromine atom, or an iodine atom.

Examples of the styrene compound represented by chemical formula (III) include styrene compounds represented by chemical formula (III-1) to chemical formula (III-7).

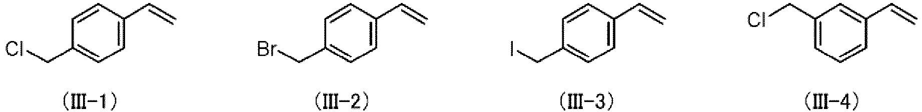

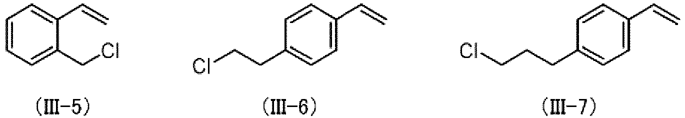

These styrene compounds for use may be commercially available reagents.

Examples of the alkyne compound represented by chemical formula (IV) include alkyne compounds represented by chemical formula (IV-1) to chemical formula (IV-4).

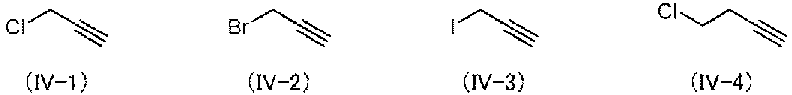

These alkyne compounds for use may be commercially available reagents or may be synthesized, for example, according to the method described in ACS Catalysis, 4(3), 722-731 (2014).

Examples of the alkene compound represented by chemical formula (V) include alkene compounds represented by chemical formula (V-1) to chemical formula (V-3).

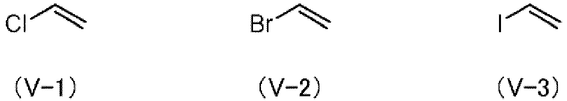

These alkene compound for use may be commercially available reagents.

The amount of the styrene compound represented by chemical formula (III), the alkyne compound represented by chemical formula (IV), or the alkene compound represented by chemical formula (V) (the amount added) is preferably an appropriate proportion within the range of 1.5-fold to 5.0-fold mol relative to the amount of the naphthalene dithiol compound represented by chemical formula (II) (the amount added).

Reaction scheme (A) shows a specific example. The naphthalene compound of the present invention represented by chemical formula (I-1-1) can be synthesized by reacting the naphthalene dithiol compound represented by chemical formula (II-1) with the styrene compound represented by chemical formula (III-1).

### Reaction scheme (A)

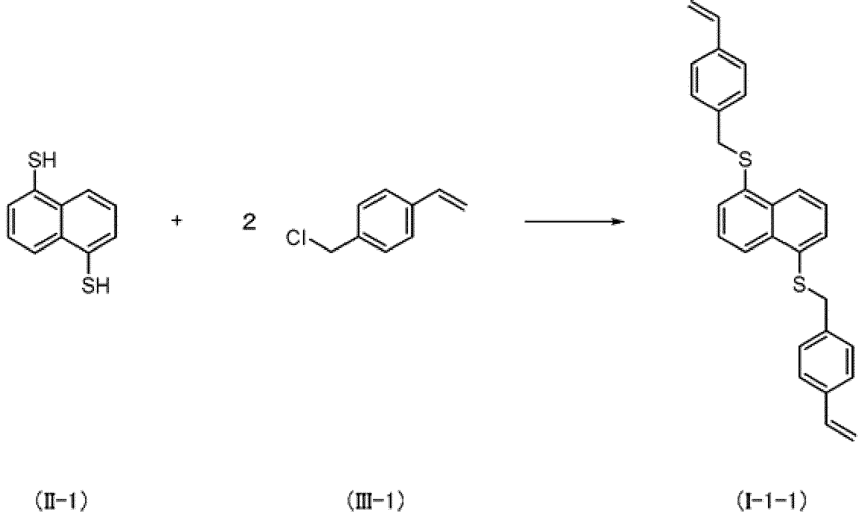

In performing this reaction, it is preferable to use a base (a) in order to remove an acid (by-product) formed over the course of reaction. A reaction solvent (b) may also be used as necessary.

Examples of the base (a) include trimethylamine, triethylamine, tributylamine, N,N-diisopropylethylamine, N-methylmorpholine, 1,5-diazabicyclo[4.3.0]non-5-ene (DBN), 1,4-diazabicyclo[2.2.2]octane (DABCO), 1,8-diazabicyclo[5.4.0]-7-undecene (DBU), pyridine, 4-(N,N-dimethylamino)pyridine, picoline, N,N-dimethylaniline, N,N-diethylaniline, imidazole, lithium hydride, sodium hydride, potassium hydride, lithium hydroxide, sodium hydroxide, potassium hydroxide, cesium hydroxide, lithium carbonate, sodium carbonate, potassium carbonate, cesium carbonate, lithium hydrogen carbonate, sodium hydrogen carbonate, potassium hydrogen carbonate, cesium hydrogen carbonate, trilithium phosphate, trisodium phosphate, tripotassium phosphate, tricesium phosphate, dilithium hydrogen phosphate, disodium hydrogen phosphate, dipotassium hydrogen phosphate, dicesium hydrogen phosphate, lithium dihydrogen phosphate, sodium dihydrogen phosphate, potassium dihydrogen phosphate, cesium dihydrogen phosphate, lithium acetate, sodium acetate, potassium acetate, cesium acetate, lithium alkoxide (e.g., lithium methoxide), sodium alkoxide (e.g., sodium methoxide and sodium ethoxide), and potassium alkoxide (e.g., potassium t-butoxide). These may be used singly or in a combination of two or more.

The amount of the base (a) (the amount added) is preferably an appropriate proportion within the range of 1.5-fold to 10.0-fold mol relative to the amount of the naphthalene dithiol compound represented by chemical formula (II) (the amount added).

The reaction solvent (b) is not particularly limited as long as the reaction solvent (b) does not interfere with the reaction. Examples of the reaction solvent (b) include solvents such as tetrahydrofuran, dioxane, ethyl acetate, acetonitrile, benzene, toluene, xylene, dichloromethane, chloroform, carbon tetrachloride, dichloroethane, dimethylformamide, dimethylacetamide, dimethylsulfoxide, hexamethylphosphoric triamide, and water. These can be combined as necessary and used in an appropriate amount.

In this reaction, the reaction temperature is preferably set within the range of 0 to 120°C. The reaction time is set as appropriate according to the predetermined reaction temperature and is preferably set within the range of 1 to 24 hours.

After completion of the reaction, the naphthalene compound of the present invention (desired product) can be taken out from the obtained reaction solution (reaction mixture) according to a means such as concentration of the reaction solution by distillation of the reaction solvent or solvent extraction.

Additionally, the naphthalene compound of the present invention can be purified according to a means such as washing with water etc., activated carbon treatment, silica gel chromatography, or recrystallization as necessary.

### Synthesis Method (2)

The naphthalene compound represented by chemical formula (I) of the present invention wherein R¹s are the same and are each a group represented by formula (3) can be synthesized by reacting a naphthalene dithiol compound represented by chemical formula (II) with a C₂-C₁₂ dihaloalkane compound to haloalkylate the two thiol groups (first step), and reacting the resulting compound with a base (second step) (see reaction scheme (B) described below as a specific example).

The C₂-C₁₂ dihaloalkane compound includes, for example, a compound in which one hydrogen atom on one terminal carbon atom of a C₂-C₁₂ alkane chain and one hydrogen atom on the other terminal carbon atom are each substituted with a halogen atom (e.g., a chlorine atom, a bromine atom, or an iodine atom). Examples include 1,2-dichloroethane, 1,2-dibromoethane, 1,2-diiodoethane, 1,3-dichloropropane, 1,3-dibromopropane, 1,3-diiodopropane, 1,4-dichlorobutane, 1,4-dibromobutane, 1,4-diiodobutane, 1,5-dichloropentane, and 1,6-dichlorohexane.

In the reaction of the first step, the amount of the dihalogen compound (the amount added) is preferably an appropriate proportion within the range of 2-fold to 100-fold mol relative to the amount of the naphthalene dithiol compound represented by chemical formula (II) (the amount added).

Reaction scheme (B) shows a specific example. The naphthalene compound of the present invention represented by chemical formula (I-3-12) can be synthesized by reacting the naphthalene dithiol compound represented by chemical formula (II-3) with a dihalogen compound (first step), and reacting the resulting compound with a base (second step).

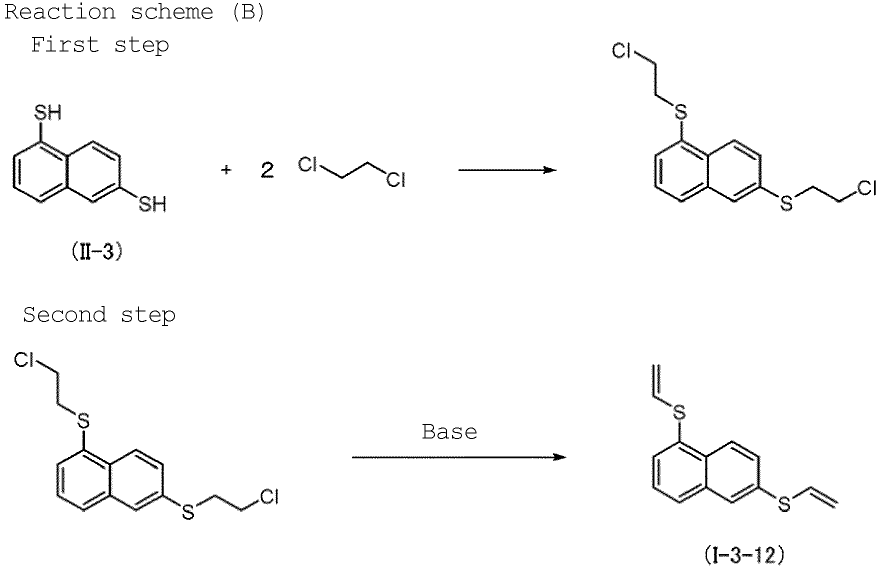

In performing the first step, it is preferable to use a base (a) in order to remove an acid (by-product) formed over the course of reaction. A reaction solvent (b) may also be used as necessary.

The base (a) and the reaction solvent (b) for use may be those listed as examples for synthesis method (1).

The amount of the base (a) (the amount added) is preferably an appropriate proportion within the range of 2-fold to 10-fold mol relative to the amount of the naphthalene dithiol compound represented by chemical formula (II) (the amount added).

In the reaction of the first step, the reaction temperature is preferably set within the range of 30 to 120°C. The reaction time is set as appropriate according to the predetermined reaction temperature and is preferably set within the range of 1 to 30 hours.

After completion of the reaction of the first step, the halogen atom-containing naphthalene compound (desired product) can be taken out from the obtained reaction solution (reaction mixture) according to a means such as concentration of the reaction solution by distillation of the reaction solvent or solvent extraction.

Additionally, the halogen atom-containing naphthalene compound can be purified according to a means such as washing with water etc., activated carbon treatment, silica gel chromatography, or recrystallization as necessary.

The halogen atom-containing naphthalene compound may be subjected to the second step after concentration, extraction, purification, etc. as mentioned above, or may be subjected to the second step directly in the form of the reaction solution obtained after the completion of the reaction of the first step.

The base for use in the second step can be those listed as examples of the base (a) for synthesis method (1).

The amount of the base (the amount added) is preferably an appropriate proportion within the range of 2-fold to 20-fold mol relative to the amount of the halogen atom-containing naphthalene compound (the amount added).

In performing the second step, a reaction solvent (b) may also be used as necessary.

The reaction solvent (b) for use can be those listed as examples for synthesis method (1).

In the reaction of the second step, the reaction temperature is preferably set within the range of -10 to 100°C. The reaction time is set as appropriate according to the predetermined reaction temperature and is preferably set within the range of 1 to 30 hours.

After completion of the reaction of the second step, the naphthalene compound of the present invention (desired product) can be taken out from the obtained reaction solution (reaction mixture) according to a means such as concentration of the reaction solution by distillation of the reaction solvent or solvent extraction.

Additionally, the naphthalene compound of the present invention can be purified according to a means such as washing with water etc., activated carbon treatment, silica gel chromatography, or recrystallization as necessary.

### 2. Composition Containing Naphthalene Compound (which may be referred to below as "the composition of the present invention")

The composition of the present invention contains the naphthalene compound of the present invention (which may be referred to below as "the first curable compound") as an essential component. The composition of the present invention may also contain one or more of the naphthalene compounds of the present invention.

The content of the naphthalene compound of the present invention in the composition of the present invention is preferably 0.001 to 100 wt%, more preferably 0.001 wt% or more and less than 100 wt%, and particularly preferably 10 wt% or more and less than 90 wt%.

Polymerizing the naphthalene compound of the present invention provides a cured product. Specifically, the composition of the present invention, which contains a naphthalene compound containing carbon-carbon unsaturated bonds (double bonds or triple bonds) in the molecule, can be considered a polymerizable or curable composition. Allowing a curable compound different from the naphthalene compound of the present invention to be present together with the naphthalene compound of the present invention during polymerization provides a cured product that is a copolymer of the naphthalene compound of the present invention with the curable compound.

Such a curable compound includes an ene compound containing a carbon-carbon double bond in the molecule (which may be referred to below as "the second curable compound"), and includes a compound containing an epoxy group, an oxetane ring, an episulfide group, or a vinyl group in the molecule (which may be referred to below as "the third curable compound").

### First Composition

The first composition of the present invention contains the naphthalene compound of the present invention as an essential component and may contain the second curable compound (an en compound containing a carbon-carbon double bond in the molecule) as necessary. In this composition, the naphthalene compound of the present invention for use is preferably a naphthalene compound in which R¹s (i) are the same or different and are each a group represented by formula (1) above, or (ii) are the same and are each a group represented by formula (2) above. For example, preferred is a compound represented by chemical formula (I-1-1), chemical formula (1-1-7), chemical formula (1-1-9), chemical formula (1-2-1), chemical formula (1-2-7), chemical formula (I-2-9), chemical formula (1-3-1), chemical formula (I-3-10), or chemical formula (I-3-12).

In terms of compatibility with various solvents and resin components, the first curable compound for use is preferably a mixture of naphthalene compounds in which R¹s are each a group represented by formula (1) above and in which the bonding position of the vinyl group in formula (1) varies (for example, a mixture of the compound represented by chemical formula (1-3-1), the compound represented by chemical formula (I-3-2), the compound represented by chemical formula (1-3-4), and the compound represented by chemical formula (I-3-7)).

The second curable compound encompasses both a polymerizable monomer and a polymerizable oligomer having the structure in which a polymerizable monomer is partially polymerized (semi-cured product).

Examples of the polymerizable monomer include
(1) (meth)acrylic acid alkyl ester monomers,
(2) hydroxy group-containing monomers,
(3) carboxyl group-containing monomers,
(4) amino group-containing monomers,
(5) acetoacetyl group-containing monomers,
(6) isocyanate group-containing monomers,
(7) glycidyl group-containing monomers,
(8) monomers containing one or more aromatic rings,
(9) monomers containing an alkoxy group and an oxyalkylene group,
(10) alkoxyalkyl (meth)acrylamide monomers,
(11) (meth)acrylamide monomers,
(12) monofunctional unsaturated compounds, and
(13) polyfunctional unsaturated compounds.

(1) Examples of the (meth)acrylic acid alkyl ester monomers include methyl (meth)acrylate, ethyl (meth)acrylate, n-butyl (meth)acrylate, iso-butyl (meth)acrylate, tert-butyl (meth)acrylate, n-propyl (meth)acrylate, n-hexyl (meth)acrylate, 2-ethylhexyl (meth)acrylate, n-octyl (meth)acrylate, isodecyl (meth)acrylate, lauryl (meth)acrylate, cetyl (meth)acrylate, stearyl (meth)acrylate, cyclohexyl (meth)acrylate, isobornyl (meth)acrylate, and polyisobornyl (meth)acrylate.
(2) Examples of the hydroxy group-containing monomers include (meth)acrylic acid hydroxyalkyl esters, such as 2-hydroxyethyl (meth)acrylate, 4-hydroxybutyl (meth)acrylate, 5-hydroxypentyl (meth)acrylate, 6-hydroxyhexyl (meth)acrylate, and 8-hydroxyoctyl (meth)acrylate; and caprolactone-modified monomers, such as caprolactone-modified 2-hydroxyethyl (meth)acrylate; and oxyalkylene-modified monomers, such as diethylene glycol (meth)acrylate and polyethylene glycol (meth)acrylate. Other examples include primary hydroxy group-containing monomers, such as 2-acryloxyethyl-2-hydroxyethyl phthalic acid, N-methylol (meth)acrylamide, and hydroxyethyl acrylamide; secondary hydroxy group-containing monomers, such as 2-hydroxypropyl (meth)acrylate, 2-hydroxybutyl (meth)acrylate, 3-chloro-2-hydroxypropyl (meth)acrylate, propylene glycol diglycidyl ether-epoxy di(meth)acrylate, phenol glycidyl ether-epoxy(meth)acrylate, and bisphenol A diglycidyl ether-epoxy di(meth)acrylate; and tertiary hydroxy group-containing monomers, such as 2,2-dimethyl 2-hydroxyethyl (meth)acrylate.
(3) Examples of the carboxyl group-containing monomers include (meth)acrylic acid, acrylic acid dimer, crotonic acid, maleic acid, maleic anhydride, fumaric acid, citraconic acid, glutaconic acid, itaconic acid, acrylamide-N-glycolic acid, and cinnamic acid.
(4) Examples of the amino group-containing monomers include tert-butylaminoethyl (meth)acrylate, ethylaminoethyl (meth)acrylate, dimethylaminoethyl (meth)acrylate, and diethylaminoethyl (meth)acrylate.
(5) Examples of the acetoacetyl group-containing monomers include 2-(acetoacetoxy)ethyl (meth)acrylate and allylacetoacetate.
(6) Examples of the isocyanate group-containing monomers include 2-acryloyloxyethyl isocyanate, 2-methacryloyloxyethyl isocyanate, and alkylene oxide adducts thereof.
(7) Examples of the glycidyl group-containing monomers include glycidyl (meth)acrylate; epoxy (meth)acrylates as reaction products of an epoxy compound and (meth)acrylic acid, such as ethylene glycol diglycidyl ether-epoxy (meth)acrylate, resorcin diglycidyl ether-epoxy (meth)acrylate, bis(4-hydroxyphenyl) sulfide diglycidyl ether-epoxy (meth)acrylate, phenolic novolac epoxy resin-(meth)acrylate, cresol novolac epoxy resin-(meth)acrylate, bisphenol (e.g., bisphenol A, bisphenol F) epoxy resin-(meth)acrylate, biphenol (e.g., 3,3',5,5'-tetramethylbiphenol) epoxy resin-(meth)acrylate, and 1,3,5-tris(2,3-epoxypropyl) isocyanurate-(meth)acrylate; and glycidyl (meth)acrylates, such as 4-hydroxybutyl (meth)acrylate glycidyl ether.
(8) Examples of the monomers containing one or more aromatic rings include phenyl (meth)acrylate, benzyl (meth)acrylate, phenoxyethyl (meth)acrylate, phenoxydiethylene glycol (meth)acrylate, 2-hydroxy-3-phenoxypropyl (meth)acrylate, naphthyl (meth)acrylate, biphenyl methyl (meth)acrylate, styrene, α-methylstyrene, and vinylnaphthalene.
(9) Examples of the monomers containing an alkoxy group and an oxyalkylene group include 2-methoxyethyl (meth)acrylate, 2-ethoxyethyl (meth)acrylate, 3-methoxybutyl (meth)acrylate, 2-butoxyethyl (meth)acrylate, 2-butoxydiethylene glycol (meth)acrylate, methoxydiethylene glycol (meth)acrylate, methoxytriethylene glycol (meth)acrylate, ethoxydiethylene glycol (meth)acrylate, methoxydipropylene glycol (meth)acrylate, methoxypolyethylene glycol (meth)acrylate, octoxypolyethylene glycol-polypropylene glycol-mono(meth)acrylate, lauroxypolyethylene glycol mono(meth)acrylate, and stearoxypolyethylene glycol mono(meth)acrylate.
(10) Examples of the alkoxyalkyl (meth)acrylamide monomers include methoxymethyl (meth)acrylamide, ethoxymethyl (meth)acrylamide, propoxymethyl (meth)acrylamide, isopropoxymethyl (meth)acrylamide, n-butoxymethyl (meth)acrylamide, and isobutoxymethyl (meth)acrylamide.
(11) Examples of the (meth)acrylamide monomers include (meth)acryloylmorpholine, dimethyl (meth)acrylamide, diethyl (meth)acrylamide, and (meth)acrylamide N-methylol (meth)acrylamide.
(12) Examples of the monofunctional unsaturated compounds include biphenyl structure-containing (meth)acrylate compounds. More specific examples include biphenyl (meth)acrylates, such as o-biphenyl (meth)acrylate, m-biphenyl (meth)acrylate, and p-biphenyl (meth)acrylate; biphenyloxyalkyl (meth)acrylates, such as o-biphenyloxymethyl (meth)acrylate, m-biphenyloxymethyl (meth)acrylate, p-biphenyloxymethyl (meth)acrylate, o-biphenyloxyethyl (meth)acrylate, m-biphenyloxyethyl (meth)acrylate, p-biphenyloxyethyl (meth)acrylate, o-biphenyloxypropyl (meth)acrylate, m-biphenyloxypropyl (meth)acrylate, and p-biphenyloxypropyl (meth)acrylate; and biphenyloxy polyalkylene glycol (meth)acrylates, such as (o-biphenyloxy)diethylene glycol (meth)acrylate, (m-biphenyloxy)diethylene glycol (meth)acrylate, (p-biphenyloxy)diethylene glycol (meth)acrylate, (o-biphenyloxy)dipropylene glycol (meth)acrylate, (m-biphenyloxy)dipropylene glycol (meth)acrylate, (p-biphenyloxy)dipropylene glycol (meth)acrylate, (o-biphenyloxy)polyethylene glycol (meth)acrylate, (m-biphenyloxy)polyethylene glycol (meth)acrylate, (p-biphenyloxy)polyethylene glycol (meth)acrylate, (o-biphenyloxy)polypropylene glycol (meth)acrylate, (m-biphenyloxy)polypropylene glycol (meth)acrylate, and (p-biphenyloxy)polypropylene glycol (meth)acrylate.
(13) Examples of the polyfunctional unsaturated compounds include bifunctional monomers, trifunctional or higher functional monomers, urethane (meth)acrylates, polyurethane (meth)acrylates, thiourethane (meth)acrylates, polythiourethane (meth)acrylates, the epoxy (meth)acrylates mentioned above, ester (meth)acrylates, polyester (meth)acrylates, polyether (meth)acrylates, (meth)acrylates with a cardo structure, sulfur-containing (meth)acrylates, and vinylthioethers.

Specific examples of bifunctional monomers include ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, dipropylene glycol di(meth)acrylate, tripropylene glycol di(meth)acrylate, polypropylene glycol di(meth)acrylate, butylene glycol di(meth)acrylate, neopentyl glycol di(meth)acrylate, ethylene oxide-modified bisphenol A di(meth)acrylate, propylene oxide-modified bisphenol A di(meth)acrylate, 1,6-hexanediol di(meth)acrylate, 1,6-hexanediol ethylene oxide-modified di(meth)acrylate, glycerin di(meth)acrylate, pentaerythritol di(meth)acrylate, ethylene glycol diglycidyl ether di(meth)acrylate, diethylene glycol diglycidyl ether di(meth)acrylate, phthalic acid diglycidyl ester di(meth)acrylate, hydroxypivalic acid-modified neopentyl glycol di(meth)acrylate, isocyanuric acid ethylene oxide-modified diacrylate, and 2-(meth)acryloyloxyethyl acid phosphate diester.

Specific examples of trifunctional or higher functional monomers includes trimethylolpropane tri(meth)acrylate, ditrimethylolpropane tetra(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, dipentaerythritol hexa(meth)acrylate, tri(meth)acryloyloxy ethoxy trimethylolpropane, glycerin polyglycidyl ether poly(meth)acrylate, tris(2-(meth)acryloyloxyethyl)isocyanurate, isocyanuric acid ethylene oxide-modified tri(meth)acrylate, ethylene oxide-modified dipentaerythritol penta(meth)acrylate, ethylene oxide-modified dipentaerythritol hexa(meth)acrylate, ethylene oxide-modified pentaerythritol tri(meth)acrylate, ethylene oxide-modified pentaerythritol tetra(meth)acrylate, and succinic acid-modified pentaerythritol tri(meth)acrylate.

Specific examples of (meth)acrylates with a cardo structure include a compound represented by chemical formula (VI) . 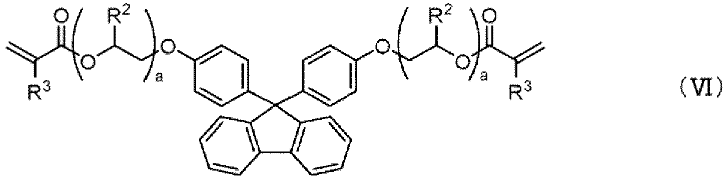 In the formula, R²s and R³s are the same or different and are each a hydrogen atom or a methyl group, and a's are the same or different and are each an integer of 1 to 4.

Specific examples of sulfur-containing (meth)acrylates include a compound represented by chemical formula (VII). 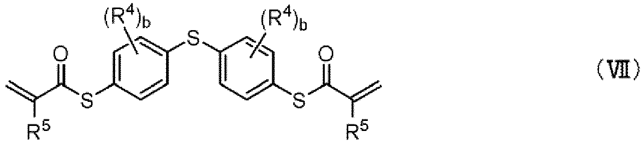 In the formula, R⁴s are the same or different and are each a C₁-C₅ alkyl group, R⁵s are the same or different and are each a hydrogen atom or a methyl group, and b's are the same or different and are each 0 or 1.

In addition to the above, examples of the polymerizable monomer also include divinylbenzene, piperylene, isoprene, pentadiene, vinylcyclohexene, chloroprene, butadiene, methylbutadiene, cyclopentadiene, methylpentadiene, acrylonitrile, methacrylonitrile, vinyl acetate, vinyl propionate, vinyl stearate, vinyl chloride, vinylidene chloride, alkyl vinyl ether, vinyl toluene, vinyl pyridine, vinyl pyrrolidone, dialkyl itaconate, dialkyl fumarate, allyl alcohol, acryloyl chloride, methyl vinyl ketone, N-acrylamidomethyltrimethylammonium chloride, allyltrimethylammonium chloride, dimethylallyl vinyl ketone, 2-chloroethyl vinyl ether, triallyl isocyanurate, tetraallyl glycoluril, N-vinylpyrrolidone, N-vinylcaprolactam, ethylene glycol diallyl carbonate, trimellitic acid triallyl ester, trifluoroethyl (meth)acrylate, tribromobenzyl (meth)acrylate, perfluorooctylethyl (meth)acrylate, sulfur-containing polyfunctional (meth)acrylate, (meth)acryloyloxypropyl tris(methoxy)silane, and styryl group-containing thiophene compounds (e.g., (3-vinylbenzyl)-2-thiophenecarboxylate and (4-vinylbenzyl)-2-thiophenecarboxylate).

The first composition of the present invention contains the naphthalene compound of the present invention as an essential component and may contain the second curable compound described above as necessary. This second curable compound for use may be a combination of the polymerizable monomer and polymerizable oligomer described above. The polymerizable monomer for use may be a combination of the polymerizable monomers described as examples above (which may be a combination of different types of polymerizable monomers), and the polymerizable oligomer for use may be a combination of different types of polymerizable oligomers.

In regards to the proportion of the content of the naphthalene compound of the present invention and the content of the second curable compound in the first composition of the present invention, the content of the second curable compound is preferably an appropriate proportion within the range of a 0-fold to 1000-fold amount (weight ratio), and more preferably an appropriate proportion within the range of a 0.01-fold to 100-fold amount (weight ratio) relative to the content of the naphthalene compound of the present invention.

The first composition of the present invention may contain a thiol compound as a curing agent. Examples of thiol compounds include aliphatic thiol compounds, such as ethanedithiol, propanedithiol, hexamethylenedithiol, decamethylenedithiol, tolylene-2,4-dithiol, 2,2-bis(mercaptomethyl)-1,3-propanedithiol, 2-(mercaptomethyl)-2-methyl-1,3-propanedithiol, and 2-ethyl-2-(mercaptomethyl)-1,3-propanedithiol; aromatic thiol compounds, such as benzenedithiol, toluenedithiol, and xylenedithiol (p-xylenedithiol); cyclic sulfide compounds, such as a 1,4-dithiane ring-containing polythiol compound represented by chemical formula (VIII); mercaptoalkylsulfide compounds, such as 3-thiapentane-1,5-dithiol and 4-mercaptomethyl-3,6-dithia-1,8-octanedithiol; mercaptopropionic acid esters, such as trimethylolpropane tris(3-mercaptopropionate) and pentaerythritol tetrakis(3-mercaptopropionate); epoxy resin-terminated mercapto compounds; mercaptoalkyl ether compounds, such as 3,6-dioxa-1,8-octanedithiol, pentaerythritol tripropanethiol, 1,2,3-(3-mercaptopropyloxy)propane, a mercaptoalkyl ether disulfide compound represented by chemical formula (IX), 2,2'-[[2,2-bis[(2-mercaptoethoxy)methyl]-1,3-propanediyl]bis(oxy)]bisethanethiol, 3,3'-[[2,2-bis[(3-mercaptopropoxy)methyl]-1,3-propanediyl]bis(oxy)]bis-1-propanethiol, 3-[2,2-bis[(3-mercaptopropoxy)methyl]butoxy]-1-propanethiol, 3-(3-mercaptopropoxy)-2,2-bis[(3-mercaptopropoxy)methyl]-1-propanol, and 2,2-bis[(3-mercaptopropoxy)methyl]-1-butanol; and 1,3,4,6-tetrakis(2-mercaptoethyl)glycoluril and 1,3,4,6-tetrakis(3-mercaptopropyl)glycoluril. These may be used in combination. 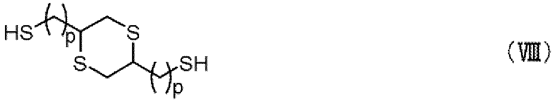 In the formula, p's are each an integer of 1 to 5. 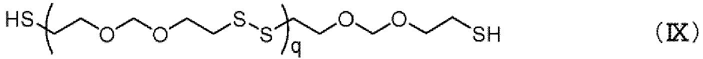 In the formula, q is an integer of 1 to 20.

In the first composition of the present invention, the content of the thiol compound is preferably 0.1 to 100 parts by weight, and more preferably 0.5 to 20 parts by weight, per 100 parts by weight of the curable compound (the sum of the first curable compound and the second curable compound).

The first composition of the present invention may also contain a reactive diluent. In the present specification, "reactive diluent" refers to a compound that contains one epoxy group (glycidyl group) and has a relatively low viscosity at an ordinary temperature.

In addition to the epoxy group, the reactive diluent may also contain other polymerizable functional groups, according to the purpose. For example, alkenyl groups, such as vinyl and allyl, and unsaturated carboxylic acid residues, such as acryloyl and methacryloyl, may be contained.

Examples of the reactive diluent include monoepoxide compounds, such as n-butyl glycidyl ether, 2-ethylhexyl glycidyl ether, phenyl glycidyl ether, cresyl glycidyl ether, p-s-butylphenyl glycidyl ether, styrene oxide, and α-pinene oxide; and monoepoxide compounds containing other functional groups, such as allyl glycidyl ether, glycidyl methacrylate, and 1-vinyl-3,4-epoxycyclohexane.

The content of the reactive diluent in the first composition of the present invention is preferably 1 to 400 parts by weight per 100 parts by weight of the curable compound (the sum of the first curable compound and the second curable compound).

The method for polymerizing (curing) the first composition of the present invention includes light curing and heat curing.

The light curing method includes a method of irradiation with active energy rays, and preferably a method that uses a photopolymerization initiator in combination. Active energy rays include light, radioactive rays, electromagnetic waves, electron beams, and the like. Preferred are electron beams or light in the ultraviolet to infrared wavelength range. For example, the light source for use for ultraviolet irradiation may be an ultra-high-pressure mercury light source or a metal halide light source, the light source for use for visible light irradiation may be a metal halide light source or a halogen light source, and the light source for use for infrared irradiation may be a halogen light source. Light sources that have been increasingly used recently, such as lasers and LEDs that emit light at various wavelengths, may also be used. The irradiation amount of active energy rays can be appropriately set, for example, according to the type of the light source.

The photopolymerization initiator can be selected from photo-radical polymerization initiators, photo-anionic polymerization initiators, and photo-cationic polymerization initiators, and they may be added to the composition. In light curing, the means of thermal polymerization (heat curing) may be used in combination to improve production efficiency and the characteristics of the cured product.

The photo-radical polymerization initiator for use can be any commonly used photo-radical polymerization initiator, without any particular limitation. Examples include acetophenones, such as acetophenone, diethoxyacetophenone, 2,2-dimethoxy-2-phenylacetophenone, 2,2-diethoxy-2-phenylacetophenone, 1,1-dichloroacetophenone, 2-hydroxy-2-methyl-1-phenylpropan-1-one, acetophenone dimethyl ketal, benzyl dimethyl ketal, 4-(2-hydroxyethoxy)phenyl-(2-hydroxy-2-propyl)ketone, 1-hydroxycyclohexylphenyl ketone, 2-methyl-2-morpholino(4-thiomethylphenyl)propan-1-one, 2-benzyl-2-dimethylamino-1-(4-morpholinophenyl)butanone, and 2-hydroxy-2-methyl-1-[4-(1-methylvinyl)phenyl]propanone oligomer; benzoins, such as benzoin, benzoin methyl ether, benzoin ethyl ether, benzoin propyl ether, benzoin isopropyl ether, and benzoin isobutyl ether; benzophenones, such as benzophenone, hydroxybenzophenone, methyl o-benzoylbenzoate, 4-phenylbenzophenone, 4-benzoyl-4'-methyl-diphenylsulfide, 4,4'-bis(methylamino)benzophenone, 3,3',4,4'-tetra(t-butylperoxycarbonyl)benzophenone, 2,4,6-trimethylbenzophenone, 4-benzoyl-N,N-dimethyl-N-[2-(1-oxo-2-propenyloxy)ethyl]benzenemethanaminium bromide, and (4-benzoylbenzyl)trimethylammonium chloride; thioxanthones, such as 2-isopropylthioxanthone, 4-isopropylthioxanthone, 2,4-diethylthioxanthone, 2,4-dichlorothioxanthone, 1-chloro-4-propoxythioxanthone, and 2-(3-dimethylamino-2-hydroxy)-3,4-dimethyl-9H-thioxanthon-9-one mesochloride; acylphosphine oxides, such as 2,4,6-trimethylbenzoyl-diphenylphosphine oxide, bis(2,6-dimethoxybenzoyl)-2,4,4-trimethyl-pentylphosphine oxide, and bis(2,4,6-trimethylbenzoyl)-phenylphosphine oxide; and methylphenylglyoxylate. These may be used alone or in a combination of two or more.

The photo-anionic polymerization initiator for use can be any commonly used photo-anionic polymerization initiator, without any particular limitation, and examples include onium salts and carbamates.

Examples of onium salts include 1,2-diisopropyl-3-(bis(dimethylamino)methylene)guanidium 2-(3-benzoylphenyl)propionate, and 1,2-dicyclohexyl-4,4,5,5-tetramethylbiguanidium n-butyltriphenylborate. Examples of carbamates include 2-nitrophenylmethylpiperidine-1-carboxylate, 1-(anthraquinone-2-yl)ethylimidazolecarboxylate, 1-(3-(2-hydroxyphenyl)-2-propenoyl)piperidine, and 9-anthranylmethyl diethyl carbamate.

The photo-cationic polymerization initiator for use can be any commonly used photo-cationic polymerization initiator, without any particular limitation, and examples include onium salts and organic metal complexes.

Examples of onium salts include diazonium salts, sulfonium salts, and iodonium salts. Examples of organic metal complexes include iron-arene complexes, titanocene complexes, and arylsilanol-aluminum complexes.

Examples of commercially available photo-cationic polymerization initiators include Adeka Optomer SP-150 (product name) and Adeka Optomer SP-170 (product name) produced by ADEKA Corporation, UVE-1014 (product name) produced by General Electric Company, CD-1012 (product name) produced by Sartomer, and CPI-100P (product name) produced by San-Apro Ltd.

Examples of counter-anions of the photo-cationic polymerization initiators include SbF₆⁻, AsF₆⁻, B(C₆F₅)₄⁻, and PF₆⁻.

The content of the photopolymerization initiator in the first composition of the present invention is preferably 0.1 to 20 parts by weight, more preferably 0.2 to 15 parts by weight, and even more preferably 0.5 to 10 parts by weight, per 100 parts by weight of the curable compound (the sum of the first curable compound and the second curable compound).

The first composition of the present invention may also contain an auxiliary agent of the photo-radical polymerization initiator. Examples of the auxiliary agent include triethanolamine, triisopropanolamine, 4,4'-dimethylamino benzophenone (Michler's ketone), 4,4'-diethylamino benzophenone, 2-dimethylaminoethylbenzoic acid, ethyl 4-dimethylaminobenzoate, (n-butoxy)ethyl 4-dimethylaminobenzoate, isoamyl 4-dimethylaminobenzoate, 2-ethylhexyl 4-dimethylaminobenzoate, 2,4-diethylthioxanthone, and 2,4-diisopropylthioxanthone.

In addition, the first composition of the present invention may also contain a sensitizer so as to expand the sensitive wavelength range and increase sensitivity. Examples of sensitizers include benzophenone, p,p'-tetramethyldiaminobenzophenone, p,p'-tetraethylaminobenzophenone, 2-chlorothioxanthone, anthrone, 9-ethoxyanthracene, anthracene, pyrene, perylene, phenothiazine, thioxanthone, benzil, acridine orange, benzoflavin, cetoflavin-T, 9,10-diphenylanthracene, 9-fluorenone, acetophenone, phenanthrene, 2-nitrofluorene, 5-nitroacenaphthene, benzoquinone, 2-chloro-4-nitroaniline, N-acetyl-p-nitroaniline, p-nitroaniline, N-acetyl-4-nitro-1-naphthylamine, picramide, anthraquinone, 2-ethylanthraquinone, 2-tert-butylanthraquinone, 1,2-benzanthraquinone, 3-methyl-1,3-diaza-1,9-benzanthrone, dibenzalacetone, 1,2-naphthoquinone, 3,3'-carbonyl-bis(5,7-dimethoxycarbonylcoumarin), and coronene.

The content of the sensitizer in the first composition of the present invention is, for example, preferably 0.1 to 20 parts by weight, more preferably 0.2 to 15 parts by weight, and even more preferably 0.5 to 10 parts by weight, per 100 parts by weight of the curable compound (the sum of the first curable compound and the second curable compound).

The method for heat-curing the first composition of the present invention includes a method that uses a thermal polymerization initiator in combination. The thermal polymerization initiator can be selected from thermal radical polymerization initiators, thermal anionic polymerization initiators, and thermal cationic polymerization initiators, and they may be added to the composition.

For the conditions of heat curing, the heating temperature and heating time can be appropriately set. The heating temperature and heating time are preferably set within the range of 60 to 130°C and the range of 30 to 240 minutes, and more preferably within the range of 70 to 125°C and the range of 30 to 120 minutes.

Examples of heating means include methods such as hot air circulation, infrared heating, and high-frequency heating. The curing device for use may be a closed curing furnace, a tunnel furnace, which allows for continuous curing, and the like.

The thermal radical polymerization initiator for use can be any commonly used thermal radical polymerization initiator, without any particular limitation. Examples include peroxides, such as diisopropyl peroxydicarbonate, benzoyl peroxide, t-butyl peroxyisobutyrate, t-hexyl peroxyisopropyl monocarbonate, t-hexylperoxy 2-ethylhexanoate, 1,1,3,3-tetramethylbutylperoxy 2-ethylhexanoate, t-butylperoxypivalate, t-hexyl peroxy pivalate, t-butyl peroxy neodecanoate, t-hexyl peroxy neodecanoate, 1,1,3,3-tetramethylbutylperoxyneodecanoate, 1,1-bis(t-hexylperoxy)cyclohexane, benzoyl peroxide, 3,5,5-trimethylhexanoyl peroxide, and lauroyl peroxide; and azo compounds, such as azobisisobutyronitrile, 2,2'-azobis(2-methylbutyronitrile), and dimethyl 2,2'-azobis(2-methylpropionate). These may be used in combination.

The thermal anionic polymerization initiator for use can be any commonly used thermal anionic polymerization initiator, without any particular limitation. Examples include amines and imidazoles. These may be used in combination.

Examples of amines include diethylenetriamine, triethylenetetramine, isophoronediamine, xylylenediamine, diaminodiphenylmethane, and 1,3,4,6-tetrakis(3-aminopropyl)glycoluril. Examples of imidazoles include 2-methylimidazole, 2-ethyl-4-methylimidazole, and 2-phenylimidazole.

The thermal cationic polymerization initiator for use can be any commonly used thermal cationic polymerization initiator, without any particular limitation. Examples include various onium salts, such as quaternary ammonium salts, phosphonium salts, and sulfonium salts, and organic metal complexes. These may be used in combination.

Examples of commercially available onium salts include Adeka Opton CP-66 (product name) and Adeka Opton CP-77 (product name) produced by ADEKA Corporation, SunAid SI-60L (product name), SunAid SI-80L (product name), and SunAid SI-100L (product name) produced by Sanshin Chemical Industry Co., Ltd., and CI series (product name) produced by Nippon Soda Co., Ltd.

Examples of organic metal complexes include alkoxysilane-aluminum complexes.

The content of the thermal polymerization initiator in the first composition of the present invention is preferably 0.001 to 20 wt%, and more preferably 0.01 to 10 wt%, based on the entire composition (total amount).

As long as the effects of the present invention are not impaired, the first composition of the present invention may further contain the following: pigments (e.g., titanium white, cyanine blue, watching red, colcothar, carbon black, aniline black, manganese blue, iron black, ultramarine blue, Hansa red, chrome yellow, and chrome green); inorganic fillers (calcium carbonate, kaolin, clay, talc, mica, barium sulfate, lithopone, gypsum, zinc stearate, oxides, such as perlite, quartz, quartz glass, silica powder such as molten silica and spherical silica, spherical alumina, crushed alumina, magnesium oxide, beryllium oxide, titanium oxide, and zirconium oxide, nitrides, such as boron nitride, silicon nitride, and aluminum nitride, carbides, such as silicon carbide, hydroxides, such as aluminum hydroxide and magnesium hydroxide, metals and alloys, such as copper, silver, iron, aluminum, nickel, titanium, platinum, and gold, carbon materials, such as diamond and carbon); thermoplastic resin and thermosetting resin (homopolymers, such as high-density, medium-density, or low-density polyethylene, polypropylene, polybutene, and polypentene, ethylene-propylene copolymers, polyamide resin, such as nylon-6 and nylon-6,6, vinyl chloride resin, nitrocellulose resin, vinylidene chloride resin, acrylic resin (including curable compounds, except for the polymerizable monomers described above), acrylamide resin, styrene resin, vinyl ester resin, polyester resin, phenolic resin (phenol compound), epoxy resin (epoxy compound), silicone resin, fluorine resin, elastomer resin, such as acrylic rubber and urethane rubber, and graft copolymers, such as methyl methacrylate-butadiene-styrene graft copolymers, and acrylonitrile-butadiene-styrene graft copolymers); reinforcing agents (e.g., glass fiber and carbon fiber); anti-drip agents (e.g., hydrogenated castor oil and particulate silicic anhydride); delusterants (e.g., fine powder silica and paraffin wax); grinding agents (e.g., zinc stearate); internal mold release agents (e.g., fatty acids, such as stearic acid, fatty acid metal salts, such as calcium stearate, fatty acid amides, such as stearic acid amide, fatty acid esters, polyolefin wax, and paraffin wax); diluents (e.g., organic solvents, such as n-butyl alcohol, methyl ethyl ketone (MEK), propylene glycol monomethyl ether acetate (PGMEA), and toluene, water, and combinations of an organic solvent with water); coupling agents (e.g., silane coupling agents, such as N-(2-aminoethyl)-3-aminopropyltrimethoxysilane and 3-glycidoxypropyltrimethoxysilane); chain transfer agents (e.g., thiol compounds, such as pentaerythritol tetrakis(3-mercaptopropionate)); and additives (modifiers), such as surfactants, leveling agents, antifoaming agents, fragrances, flame retardants, and dyes.

The thermoplastic resin and thermosetting resin encompass resins (compounds) with a cardo structure represented by chemical formula (X) (a skeleton structure in which four aromatic rings are bound to a carbon atom). 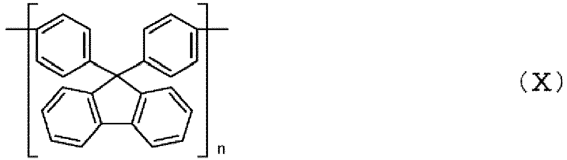 In the formula, n indicates the degree of polymerization.

Examples of compounds with a cardo structure include monomers, such as 9,9-bis(4-glycidyloxyphenyl)fluorene, 9,9-bis(4-hydroxyphenyl)fluorene, 9,9-bis(4-hydroxy-3-methylphenyl)fluorene, 9,9-bis[4-(2-(meth)acryloyloxyethoxy)phenyl]fluorene, 9,9-bis[4-(3-(meth)acryloyloxy-2-hydroxypropoxy)phenyl]fluorene, 9,9-bis(cyanomethyl)fluorene, and 9,9-bis(3-aminopropyl)fluorene.

In the first composition of the present invention, the content of additives (modifiers), if contained, may be 0.01 to 85 wt% based on the entire composition (total amount).

Table 1 shows a preferable example of the formulation of the first composition of the present invention (except for organic solvents).

**Table 1**

| | | Preferable amount (parts by weight) |
|---|---|---|
| (i) First curable compound | | 100 |
| | Naphthalene compound of the present invention | |
| (ii) Second curable compound | | 0-500 |
| | (Meth)acrylates with a cardo structure, sulfur-containing (meth)acrylates, styryl group-containing thiophene compounds, monomers containing one or more aromatic rings, or (meth)acrylic acid alkyl ester monomers | |
| (iii) Thiol compound | | 0-10 |
| (iv) Photo-radical polymerization initiator | | 0.1-20 |
| | 1-Hydroxycyclohexylphenyl ketone or bis(2,4,6-trimethylbenzoyl)-phenylphosphine oxide | |
| (v) Inorganic filler | | 0-500 |
| | Zirconium oxide, titanium oxide, platinum, or gold | |

The method for preparing the first composition of the present invention is not particularly limited. For example, the first composition of the present invention can be prepared by mixing the naphthalene compound of the present invention, the second curable compound, a photopolymerization initiator and/or a thermal polymerization initiator, and additives (modifiers), or by mixing a solution or dispersion of the naphthalene compound of the present invention in a diluent (organic solvent) and/or a reactive diluent, the second curable compound, a photopolymerization initiator and/or a thermal polymerization initiator, and additives (modifiers). Mixing means for use may be any known method.

### Second Composition

The second composition of the present invention contains the naphthalene compound of the present invention as an essential component and may contain a third curable compound (a compound containing an epoxy group, an oxetane ring, an episulfide group, or a vinyl group in the molecule) as necessary. In this composition, the naphthalene compound of the present invention for use is preferably a naphthalene compound in which R¹s (iii) are the same and are each a group represented by formula (3) above.

The third curable compound encompasses both a polymerizable monomer and a polymerizable oligomer having the structure in which a polymerizable monomer is partially polymerized (semi-cured product).

Examples of the polymerizable monomer include known epoxy compounds (note: sometimes called "epoxy resins"), oxetane compounds, epoxy-oxetane compounds (containing an oxirane ring and an oxetane ring in the molecule), episulfide compounds, and vinyl monomers.

The epoxy compounds for use can be any epoxy compound containing an oxirane ring (epoxy group/glycidyl group) in the molecule, without any particular limitation. Examples include polyglycidyl ethers obtained by reacting epichlorohydrin with polyhydric phenols, such as bisphenol A, bisphenol F, bisphenol AD, catechol, and resorcinol, or polyhydric alcohols, such as glycerol and polyethylene glycol; glycidyl ether esters obtained by reacting epichlorohydrin with hydroxycarboxylic acids, such as p-hydroxybenzoic acid and β-hydroxynaphthoic acid; polyglycidyl esters obtained by reacting epichlorohydrin with polycarboxylic acids, such as phthalic acid and terephthalic acid; glycidyl glycoluril compounds containing two or more epoxy groups in the molecule, such as 1,3,4,6-tetraglycidyl glycoluril; alicyclic epoxy compounds, such as 3',4'-epoxycyclohexylmethyl 3,4-epoxycyclohexanecarboxylate and bicyclononadiene diepoxide; nitrogen-containing cyclic epoxy compounds, such as triglycidyl isocyanurate and hydantoin-based epoxy compounds; and epoxidized phenol novolac resins, epoxidized cresol novolac resins, epoxidized polyolefins, cycloaliphatic epoxy resins, and urethane-modified epoxy resins. Examples also include epoxy-modified organopolysiloxane compounds produced by hydrosilylation addition reaction of silicon compounds containing an SiH group with organic compounds containing a carbon-carbon double bond and a glycidyl group (e.g., the epoxy-modified organopolysiloxane compounds disclosed in JP2004-99751A and JP2006-282988A). These may be used in combination.

The oxetane compounds for use can be any oxetane compound containing an oxetane ring (oxetanyl group/oxetane group) in the molecule, without any particular limitation. Examples include 3-ethyl-3-hydroxymethyloxetane, 3-(meth)allyloxymethyl-3-ethyloxetane, (3-ethyl-3-oxetanylmethoxy)methylbenzene, 4-fluoro-[1-(3-ethyl-3-oxetanylmethoxy)methyl]benzene, 4-methoxy-[1-(3-ethyl-3-oxetanylmethoxy)methyl]benzene, [1-(3-ethyl-3-oxetanylmethoxy)ethyl]phenyl ether, isobutoxymethyl (3-ethyl-3-oxetanylmethyl)ether, isobornyloxyethyl (3-ethyl-3-oxetanylmethyl)ether, isobornyl (3-ethyl-3-oxetanylmethyl)ether, 2-ethylhexyl (3-ethyl-3-oxetanylmethyl)ether, ethyl diethylene glycol (3-ethyl-3-oxetanylmethyl)ether, dicyclopentadiene (3-ethyl-3-oxetanylmethyl)ether, dicyclopentenyloxyethyl (3-ethyl-3-oxetanylmethyl)ether, dicyclopentenyl (3-ethyl-3-oxetanylmethyl)ether, tetrahydrofurfuryl (3-ethyl-3-oxetanylmethyl)ether, 2-hydroxyethyl (3-ethyl-3-oxetanylmethyl)ether, 2-hydroxypropyl (3-ethyl-3-oxetanylmethyl)ether, butoxyethyl (3-ethyl-3-oxetanylmethyl)ether, bornyl (3-ethyl-3-oxetanylmethyl)ether, 3,7-bis(3-oxetanyl)-5-oxa-nonane, 3,3'-(1,3-(2-methylenyl)propanediyl bis(oxymethylene))bis-(3-ethyloxetane), 1,4-bis[(3-ethyl-3-oxetanylmethoxy)methyl]benzene, 1,2-bis[(3-ethyl-3-oxetanylmethoxy)methyl]ethane, 1,3-bis[(3-ethyl-3-oxetanylmethoxy)methyl]propane, ethylene glycol bis(3-ethyl-3-oxetanylmethyl)ether, dicyclopentenyl bis(3-ethyl-3-oxetanylmethyl)ether, triethylene glycol bis(3-ethyl-3-oxetanylmethyl)ether, tetraethylene glycol bis(3-ethyl-3-oxetanylmethyl)ether, tricyclodecanediyl dimethylene (3-ethyl-3-oxetanylmethyl)ether, trimethylolpropane tris(3-ethyl-3-oxetanylmethyl)ether, 1,4-bis(3-ethyl-3-oxetanylmethoxy)butane, 1,6-bis(3-ethyl-3-oxetanylmethoxy)hexane, pentaerythritol tris(3-ethyl-3-oxetanylmethyl)ether, pentaerythritol tetrakis(3-ethyl-3-oxetanylmethyl)ether, polyethylene glycol bis(3-ethyl-3-oxetanylmethyl)ether, dipentaerythritol hexakis(3-ethyl-3-oxetanylmethyl)ether, dipentaerythritol pentakis(3-ethyl-3-oxetanylmethyl)ether, dipentaerythritol tetrakis(3-ethyl-3-oxetanylmethyl)ether, caprolactone-modified dipentaerythritol hexakis(3-ethyl-3-oxetanylmethyl)ether, caprolactone-modified dipentaerythritol pentakis(3-ethyl-3-oxetanylmethyl)ether, ditrimethylolpropane tetrakis(3-ethyl-3-oxetanylmethyl)ether, EO-modified bisphenol A bis(3-ethyl-3-oxetanylmethyl)ether, PO-modified bisphenol A bis (3-ethyl-3-oxetanylmethyl)ether, EO-modified hydrogenated bisphenol A bis(3-ethyl-3-oxetanylmethyl)ether, PO-modified hydrogenated bisphenol A bis(3-ethyl-3-oxetanylmethyl)ether, and EO-modified bisphenol F (3-ethyl-3-oxetanylmethyl)ether.

The epoxy-oxetane compounds for use can be any epoxy-oxetane compound containing an oxirane ring (same as above) and an oxetane ring (same as above), without any particular limitation. Examples include those described in U.S. Patent No. 3457193, JP2005-002191A, JP2007-270070A, JP2010-111713A, or JP2011-208089A. The epoxy-oxetane compounds described in these references are included herein by reference.

The episulfide compounds for use can be any episulfide compound containing an episulfide group in the molecule, without any particular limitation. Examples include episulfide compounds obtained by converting some or all of the epoxy groups in the epoxy compounds described above into episulfide groups.

Other examples include thiiranemethanethiol, 2,2'-bis[9H-fluoren-9-ylidene bis(6,2-phenyleneoxymethylene)]thiirane, and 2,2'-bis[9H-fluoren-9-ylidenebis(6,2-naphthyleneoxymethylene)]thiirane; examples of episulfide compounds containing an unsaturated group include vinylphenyl thioglycidyl ether, vinylbenzyl thioglycidyl ether, thioglycidyl methacrylate, thioglycidyl acrylate, and allyl thioglycidyl ether.

The vinyl monomers for use can be any vinyl monomer that is capable of cationic polymerization, without any particular limitation. Examples include styrenes, alkenyl ethers, indene, and N-vinylcarbazole. Preferred are styrenes and alkenyl ethers.

Examples of styrenes include styrene, o-methylstyrene, m-methylstyrene, p-methylstyrene, o-methoxystyrene, m-methoxystyrene, p-methoxystyrene, o-chlorostyrene, m-chlorostyrene, and p-chlorostyrene.

Examples of alkenyl ethers include alkyl vinyl ethers, such as methyl vinyl ether, ethyl vinyl ether, propyl vinyl ether, isopropyl vinyl ether, n-butyl vinyl ether, sec-butyl vinyl ether, tert-butyl vinyl ether, isobutyl vinyl ether, n-amyl vinyl ether, and isoamyl vinyl ether;
fluoroalkyl vinyl ethers, such as trifluoromethyl vinyl ether, pentafluoroethyl vinyl ether, and 2,2,2-trifluoroethyl vinyl ether; alkoxyalkyl vinyl ethers, such as 2-methoxyethyl vinyl ether, 2-ethoxyethyl vinyl ether, 2-tetrahydropyranyl vinyl ether, and 2-tetrahydrofuranyl vinyl ether;
cycloalkyl vinyl ethers, such as cyclopentyl vinyl ether, cyclohexyl vinyl ether, cycloheptyl vinyl ether, cyclooctyl vinyl ether, 2-bicyclo[2.2.1]heptyl vinyl ether, 2-bicyclo[2.2.2]octyl vinyl ether, 8-tricyclo[5.2.1.0^{2,6}]decanyl vinyl ether, 1-adamantyl vinyl ether, and 2-adamantyl vinyl ether;
aryl vinyl ethers, such as phenyl vinyl ether, 4-methyl phenyl vinyl ether, 4-trifluoromethyl phenyl vinyl ether, and 4-fluorophenyl vinyl ether; and
arylalkyl vinyl ethers, such as benzyl vinyl ether and 4-fluorobenzyl vinyl ether.

The second composition of the present invention contains the naphthalene compound of the present invention as an essential component and may contain the third curable compound described above as necessary. The third curable compound for use may be a combination of the polymerizable monomer and polymerizable oligomer described above. The polymerizable monomer for use may be a combination of the polymerizable monomers described as examples above (which may be a combination of different types of polymerizable monomers), and the polymerizable oligomer for use may be a combination of different types of polymerizable oligomers.

In regards to the proportion of the content of the naphthalene compound of the present invention and the content of the third curable compound in the second composition of the present invention, the content of the third curable compound is preferably an appropriate proportion within the range of a 0-fold to 1000-fold amount (weight ratio), and more preferably an appropriate proportion within the range of a 0.01-fold to 100-fold amount (weight ratio) relative to the content of the naphthalene compound of the present invention.

The method for polymerizing (curing) the second composition of the present invention includes light curing and heat curing.

The light curing method includes a method of irradiation with active energy rays, and preferably a method that uses a photopolymerization initiator in combination. Active energy rays include light, radioactive rays, electromagnetic waves, electron beams, and the like. Preferred are electron beams or light in the ultraviolet to infrared wavelength range. For example, the light source for use for ultraviolet irradiation may be an ultra-high-pressure mercury light source or a metal halide light source, the light source for use for visible light irradiation may be a metal halide light source or a halogen light source, and the light source for use for infrared irradiation may be a halogen light source. Light sources that have been increasingly used recently, such as lasers and LEDs that emit light at various wavelengths, may also be used. The irradiation amount of active energy rays can be appropriately set, for example, according to the type of the light source.

The photopolymerization initiator for use may be a photo-cationic polymerization initiator, and a photo-radical polymerization initiator may be used in combination as necessary. They may be added to the composition. In light curing, the means of heat curing may be used in combination to improve production efficiency and the characteristics of the cured product.

The photo-cationic polymerization initiator for use can be any commonly used photo-cationic polymerization initiator, without any particular limitation. Examples include onium salts and organic metal complexes. The photo-cationic polymerization initiator for use may be the photo-cationic polymerization initiators listed in the section for the first composition.

The content of the photo-cationic polymerization initiator in the second composition of the present invention is preferably 0.001 to 20 wt%, and more preferably 0.01 to 10 wt%.

The photo-radical polymerization initiator for use may be the photo-radical polymerization initiators listed in the section for the first composition.

The content of the photo-radical polymerization initiator in the second composition of the present invention is preferably 0.001 to 20 wt%, and more preferably 0.01 to 10 wt%.

For light-curing the second composition of the present invention, the sensitizers listed in the section for the first composition can be used.

On the other hand, for heat-curing the second composition of the present invention, a thermal polymerization initiator can be used. The thermal polymerization initiator for use may be a thermal cationic polymerization initiator and may be added to the composition.

Examples of heating means include methods such as hot air circulation, infrared heating, and high-frequency heating. The curing device for use may be a closed curing furnace, a tunnel furnace, which allows for continuous curing, and the like.

The heating (curing) temperature and heating (curing) time may be appropriately set, taking into consideration the formulation and shape (thickness) of the composition, which is the object for irradiation.

The thermal cationic polymerization initiator for use can be any commonly used thermal cationic initiator, without any particular limitation. Examples include various onium salts, such as quaternary ammonium salts, phosphonium salts, and sulfonium salts, and organic metal complexes. The thermal cationic polymerization initiator for use may be the thermal cationic polymerization initiators listed in the section for the first composition.

The content of the thermal cationic polymerization initiator in the second composition of the present invention is preferably 0.001 to 20 wt%, and more preferably 0.01 to 10 wt%.

As long as the effects of the present invention are not impaired, the second composition of the present invention may further contain additives (modifiers) listed in the section for the first composition as necessary. The amount may be 0.01 to 85 wt% based on the entire second composition (total amount).

Table 2 shows a preferable example of the formulation of the second composition of the present invention (except for organic solvents).

**Table 2**

| | | Preferable amount (parts by weight) |
|---|---|---|
| (i) First curable compound | | 100 |
| | Naphthalene compound of the present invention | |
| (ii) Third curable compound | | 0-1000 |
| | 3',4'-Epoxycyclohexylmethyl 3,4-epoxycyclohexanecarboxylate or bicyclononadiene diepoxide | |
| (iii) Photo-cationic polymerization initiator | | 0.1-20 |
| | Onium salts (sulfonium salts) | |
| (iv) Inorganic filler | | 0-500 |
| | Zirconium oxide, titanium oxide, platinum, or gold | |

The method for preparing the second composition of the present invention is not particularly limited, and the second composition of the present invention can be prepared by measuring out the predetermined amounts of the components described above and mixing them with stirring. For example, after preliminary mixing, a roll kneader, a kneader, an extruder, or the like may be used for mixing or melt kneading for the preparation. Organic solvents (diluents for adjusting the viscosity) may also be used as necessary.

### 3. Application

The composition of the present invention (below, this composition includes "the first composition" and "the second composition") is expected to provide a cured product that is excellent in low thermal expansion properties, heat resistance, and mechanical strength, and further expected to provide a cured product with a high refractive index. Thus, the composition of the present invention is suitable as a material for use in the production of coating materials, ink, adhesives, tackifiers, gas barrier films, color filters, optical films, optical lenses, touch panels, and the like.

The coating materials are used in, for example, protection (hard coating) for touch panels, plastic containers, plastic sheets, plastic films, film liquid crystal devices, polarizers used in liquid crystal displays, optical components, or interior building materials (e.g., floor materials, wall materials, and artificial marble).

The ink is, for example, coloring ink, printing ink, UV ink, or inkjet ink. These inks are used in offset printing, flexographic printing, gravure printing, screen printing, inkjet printing, etc.

The adhesives are used in, for example, semiconductors, optics, optical components, optical waveguide coupling, optical waveguide peripheral component fixing, or CD/DVD bonding.

The tackifiers are used in, for example, adhesive (gluing) tapes, adhesive (gluing) sheets, or adhesive (gluing) labels.

The gas barrier films are used in, for example, electronic paper, flexible displays, organic EL devices, or organic solar cells.

The color filters are used in, for example, color liquid crystal displays (color filter on array (COA)), color image sensors, or organic EL displays.

The optical films are used in, for example, protective films for polarizers, films for liquid crystal displays, such as support films for prism sheets and light-guiding films, functional films, such as hard coating films, decorative films, and transparent conductive films, weather (light)-resistant films for solar cells, films for LED lighting or organic EL lighting, or transparent heat-resistant films for flexible electronics.

The optical lenses are used in, for example, lenses for microscopes, endoscopes, telescopes, cameras, glasses, etc., optical cover lenses, refractive index-distributed lenses, Fresnel lenses, lenses for smart glasses, lenticular lenses, VR lenses, AR lenses, lenses for laser beam printers, lenses for sensors, prism lenses, or pickup lenses for optical discs.

The touch panels are used in, for example, personal computers, car navigation systems, cellular phones, electronic dictionaries, or office automation or factory automation equipment.

The composition of the present invention is also expected to have applications in raw materials or components, such as transparent materials, high-refractive-index layers of anti-reflection films, reflectors, other optical thin films, core materials for optical fibers, cladding materials, optical waveguides, holograms, various other optical materials, dicing tapes, insulating materials (e.g., wire coating), solder resist ink, printed circuit boards, copper-clad laminates, copper foil with resin, prepregs, high-voltage insulating materials, interlayer insulating materials, passivation films for TFT, gate-insulating films for TFT, interlayer-insulating films for TFT, transparent planarization films for TFT, packing for insulation, insulation coating materials, paint, UV powder paint, molding materials (e.g., sheets, films, and FRP), sealing materials, liquid crystal sealing materials, sealing materials for display devices, high-heat-resistant sealing materials, potting materials, sealant materials (semiconductor sealants, sealants for electrical materials, sealants for organic EL or LED devices, and sealants for various solar cells), resist materials (liquid resist materials, color resist materials, dry film resist materials, solder resist materials, and color filter resist materials), photo spacer materials for liquid crystal cells, optical molding, materials for solar cells, materials for fuel cells, display materials, recording materials, photosensitive drums for copiers, liquid crystal displays, micro-displays, light-guiding panels for liquid crystals, micro-lens arrays, Fresnel rhomb retarders, polarizing beam splitters, materials in the electrical or electronic field, such as solid electrolytes for batteries, materials for vibration isolators, waterproof materials, moisture-proof materials, heat-shrinkable rubber tubes, O-rings, gas separation membranes, concrete protection materials, linings, soil injection agents, cooling or heat storage materials, sealing materials for sterilization equipment, and oxygen-permeable membranes; and applications in additives (modifiers) for other (thermoplastic, thermosetting, or light-curing) resins or resin compositions.

### Examples

The present invention is described in more detail below with reference to Examples and Comparative Example. However, the invention is not limited to these Examples.

The main starting materials used in the Examples and Comparative Example are as described below.

### Main Starting Material

- 1,5-Naphthalenedithiol (produced by Sugai Chemical Industry Co., Ltd.; see the compound represented by chemical formula (II-1))
- 2,6-Naphthalenedithiol (produced by Sugai Chemical Industry Co., Ltd.; see the compound represented by chemical formula (II-2))
- 1,6-Naphthalenedithiol (produced by Sugai Chemical Industry Co., Ltd.; see the compound represented by chemical formula (II-3))
- 4-Vinylbenzyl chloride (produced by Tokyo Chemical Industry Co., Ltd.; see the compound represented by chemical formula (III-1))
- A mixture of 3-vinylbenzyl chloride and 4-vinylbenzyl chloride (produced by AGC Seimi Chemical Co., Ltd., product name: CSM-P, a mixture of a compound represented by chemical formula (III-1) and a compound represented by chemical formula (III-4))
- Propargyl bromide (produced by Tokyo Chemical Industry Co., Ltd.; see the compound represented by chemical formula (IV-2))
- 1,2-Dichloroethane (produced by Tokyo Chemical Industry Co., Ltd.)
- Triethylamine (produced by Tokyo Chemical Industry Co., Ltd.)
- Potassium t-butoxide (produced by Tokyo Chemical Industry Co., Ltd.)
- Dichloromethane (produced by Wako Pure Chemical Industries, Ltd.)
- Dimethylacetamide (produced by Wako Pure Chemical Industries, Ltd.)
- Toluene (produced by Wako Pure Chemical Industries, Ltd.)
- Dimethylformamide (produced by Wako Pure Chemical Industries, Ltd.)
- Hydrochloric acid (produced by Wako Pure Chemical Industries, Ltd.)

The method for evaluation testing (measurement of refractive index) used in the Examples and Comparative Example is as described below.

### Measurement of Refractive Index

The compounds of the Examples and Comparative Example were individually dissolved in dimethylformamide (DMF) to give a concentration of 40 wt%, thereby preparing measurement samples (DMF solution) .

The prepared DMF solutions and DMF alone were measured for refractive index (25°C) in LED light (D-line wavelength) according to JIS K 0062 (Test Methods for Refractive Index of Chemical Products) with an Abbe refractometer (NAR-1T SOLID, produced by Atago Co., Ltd.).

From the obtained measurement values, the refractive index of the compounds of the Examples and Comparative Example was calculated according to the following formula. Refractive index = (refractive index of DMF solution (compound concentration: 40 wt%) - refractive index of DMF alone)/0.4 + refractive index of DMF alone

### Example 1

### Synthesis of 1,5-bis{[(4-ethenylphenyl)methyl]thio}naphthalene

961 mg (5.0 mmol) of 1,5-naphthalenedithiol, 1.83 g (12.0 mmol) of 4-vinylbenzyl chloride, and 30 mL of 1,2-dichloroethane were placed in a 100 mL flask, and 2.43 g (24.0 mmol) of triethylamine was added thereto while stirring. The mixture was then stirred at 50°C for 12 hours.

Subsequently, the mixture was cooled to room temperature, and insoluble matter was filtered off. The obtained filtrate was washed three times with 50 mL of water and concentrated under reduced pressure. The obtained concentrate was recrystallized with 10 mL of toluene to thus obtain 320 mg of white crystals (yield: 15%).

The ¹H-NMR spectral data of the obtained white crystals were as follows.
¹H-NMR (CDCl₃) δ: 8.37 (d, 2H), 7.08-7.54 (m, 12H), 6.61-6.71 (m, 2H), 5.68 (dd, 2H), 5.23 (dd, 2H), 4.13 (s, 4H).

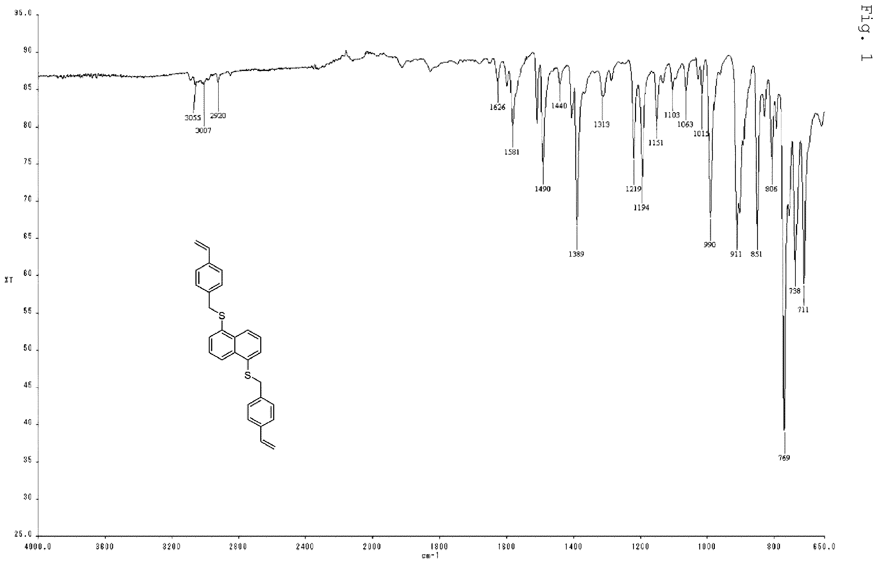

The IR spectral data of the white crystals were as shown in the chart of Fig. 1.

From these spectral data, the obtained white crystals were identified as being the title naphthalene compound represented by chemical formula (I-1-1).

### Example 2

### Synthesis of 1,5-bis(2-propyn-1-ylthio)naphthalene

961 mg (5.0 mmol) of 1,5-naphthalenedithiol, 1.19 g (10.0 mmol) of propargyl bromide, and 30 mL of dichloromethane were placed in a 100 mL flask, and 2.43 g (24.0 mmol) of triethylamine was added thereto while stirring. The mixture was then stirred at room temperature for 12 hours.

Subsequently, insoluble matter was filtered off at room temperature. The obtained filtrate was washed three times with 50 mL of water and concentrated under reduced pressure. The obtained concentrate was recrystallized with 20 mL of toluene to thus obtain 850 mg of pale yellow crystals (yield: 63%).

The ¹H-NMR spectral data of the obtained pale yellow crystals were as follows.
¹H-NMR (CDCl₃) δ: 8.42 (d, 2H), 7.79 (d, 2H), 7.52 (dd, 2H), 3.65 (d, 4H), 2.20 (t, 2H).

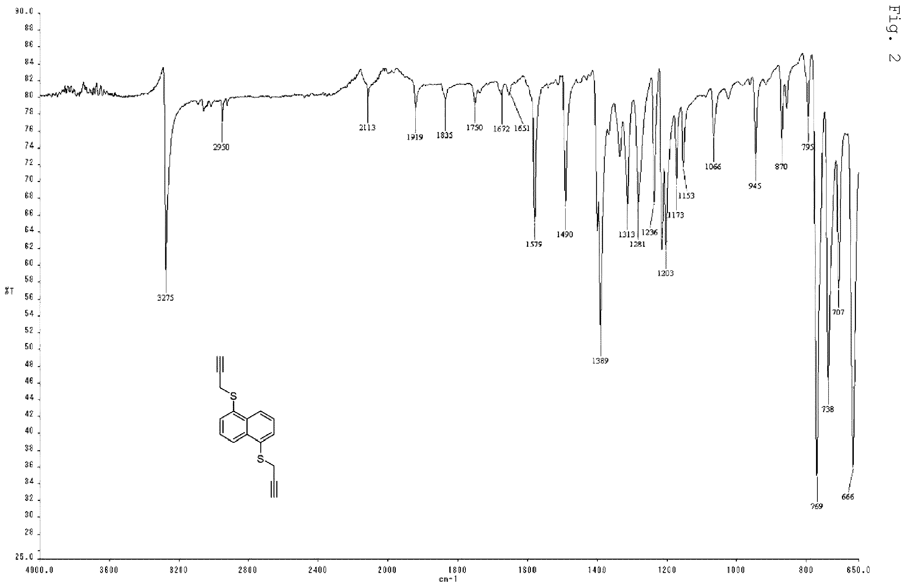

The IR spectral data of the pale yellow crystals were as shown in the chart of Fig. 2.

From these spectral data, the obtained pale yellow crystals were identified as being the title naphthalene compound represented by chemical formula (I-1-7).

### Example 3

### Synthesis of 1,5-bis(ethenylthio)naphthalene

961 mg (5.0 mmol) of 1,5-naphthalenedithiol, 19.79 g (200.0 mmol) of 1,2-dichloroethane, and 30 mL of dimethylformamide were placed in a 100 mL flask, and 1.42 g (14.0 mmol) of triethylamine was added thereto while stirring. The mixture was then heated to 60°C and stirred at the same temperature for 2 hours.

Thereafter, the mixture was cooled to room temperature, and 30 mL of toluene was added thereto. The mixture was then washed five times with 50 mL of water and concentrated under reduced pressure to thus obtain 1.21 g of a pale yellow solid.

Thereafter, 1.21 g of the obtained pale yellow solid and 19.1 mL of dimethylacetamide were placed in a 100 mL flask. The mixture was cooled to -10°C while stirring, and 1.20 g (10.7 mmol) of potassium t-butoxide was added in divided portions so as not to exceed 0°C. The mixture was then stirred at 0°C for 2 hours.

Thereafter, 20 mL of toluene was added thereto, and the mixture was washed five times with 30 mL of water and concentrated under reduced pressure to thus obtain 892 mg of a pale yellow solid (yield: 73%).

The ¹H-NMR spectral data of the obtained pale yellow solid were as follows.
¹H-NMR (CDCl₃) δ: 8.24 (d, 2H), 7.76 (d, 2H), 7.63-7.68 (m, 2H), 6.73 (dd, 2H), 5.43 (d, 2H), 5.16 (d, 2H).

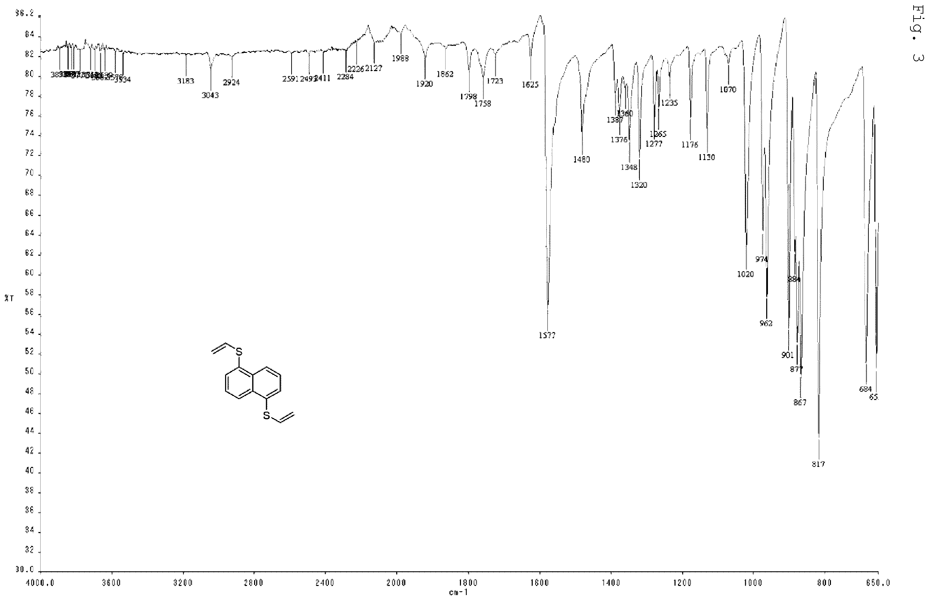

The IR spectral data of the pale yellow solid were as shown in the chart of Fig. 3.

From these spectral data, the obtained pale yellow solid was identified as being the title naphthalene compound represented by chemical formula (I-1-9).

### Example 4

### Synthesis of 2,6-bis{[(4-ethenylphenyl)methyl]thio}naphthalene

961 mg (5.0 mmol) of 2,6-naphthalenedithiol, 1.83 g (12.0 mmol) of 4-vinylbenzyl chloride, and 30 mL of dimethylacetamide were placed in a 100 mL flask, and 1.42 g (14.0 mmol) of triethylamine was added thereto while stirring. The mixture was then stirred at 50°C for 8 hours.

Subsequently, the mixture was cooled to room temperature, and insoluble matter was filtered off. The obtained filtrate was added to 80 mL of toluene, and the mixture was washed once with 40 mL of water and concentrated under reduced pressure. The obtained concentrate was poured into 150 mL of methanol to thus obtain 1.31 g of white crystals (yield: 62%).

The ¹H-NMR spectral data of the obtained white crystals were as follows.
¹H-NMR (CDCl₃) δ: 7.81 (d, 2H), 7.73 (d, 2H), 7.23-7.51 (m, 10H), 6.64-6.72 (m, 2H), 5.77 (dd, 2H), 5.22 (dd, 2H), 4.33 (s, 4H) .

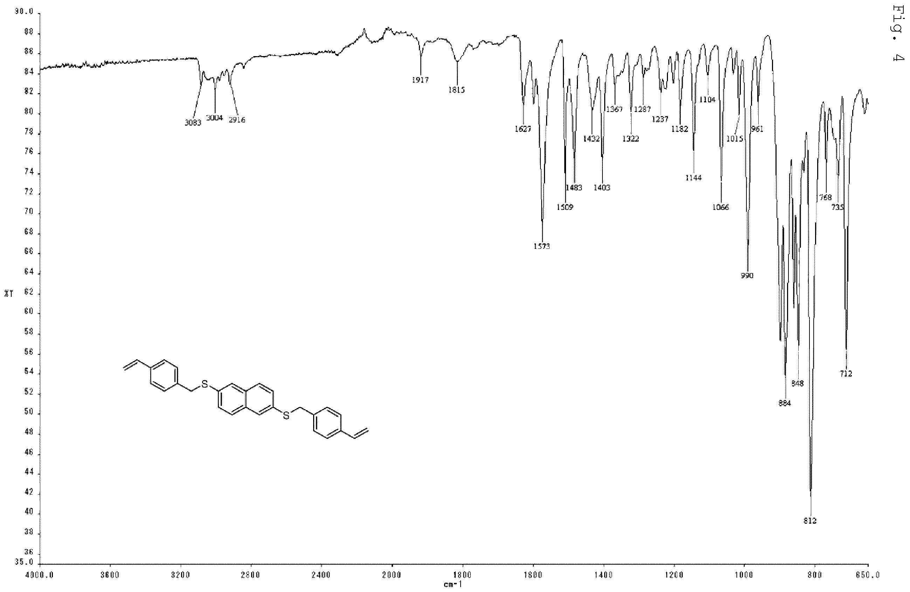

The IR spectral data of the white crystals were as shown in the chart of Fig. 4.

From these spectral data, the obtained white crystals were identified as being the title naphthalene compound represented by chemical formula (I-2-1).

### Example 5

### Synthesis of 2,6-bis(2-propyn-1-ylthio)naphthalene

961 mg (5.0 mmol) of 2,6-naphthalenedithiol, 1.19 g (10.0 mmol) of propargyl bromide, and 30 mL of dichloromethane were placed in a 100 mL flask, and 2.43 g (24.0 mmol) of triethylamine was added thereto while stirring. The mixture was then stirred at room temperature for 12 hours.

Subsequently, insoluble matter was filtered off at room temperature. The obtained filtrate was washed three times with 50 mL of water and concentrated under reduced pressure. The obtained concentrate was recrystallized with 20 mL of toluene to thus obtain 790 mg of pale yellow crystals (yield: 59%).

The ¹H-NMR spectral data of the obtained pale yellow crystals were as follows.
¹H-NMR (CDCl₃) δ: 8.87 (d, 2H), 7.75 (d, 2H), 7.52 (dd, 2H), 3.72 (d, 4H), 2.54 (t, 2H).

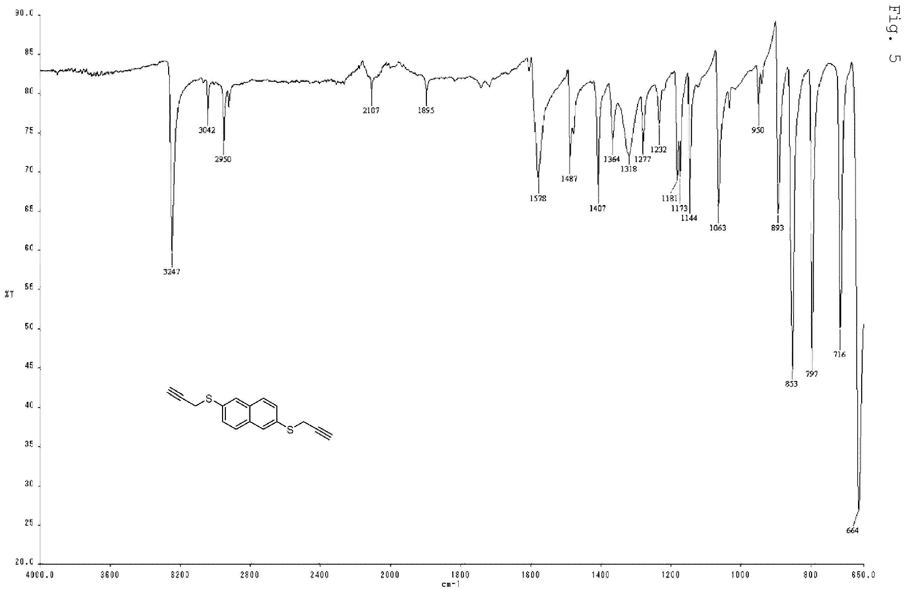

The IR spectral data of the pale yellow crystals were as shown in the chart of Fig. 5.

From these spectral data, the obtained pale yellow crystals were identified as being the title naphthalene compound represented by chemical formula (I-2-7).

### Example 6

### Synthesis of 1,6-bis{[(4-ethenylphenyl)methyl]thio}naphthalene

961 mg (5.0 mmol) of 1,6-naphthalenedithiol, 1.56 g (10.2 mmol) of 4-vinylbenzyl chloride, and 30 mL of dichloromethane were placed in a 100 mL flask, and 2.02 g (20.0 mmol) of triethylamine was added thereto while stirring. The mixture was then stirred at room temperature for 12 hours.

Subsequently, insoluble matter was filtered off at room temperature. The obtained filtrate was washed three times with 50 mL of water and concentrated under reduced pressure. The obtained concentrate was washed with 30 mL of 2-propanol to thus obtain 590 mg of a pale yellow liquid (yield: 28%).

The ¹H-NMR spectral data of the obtained pale yellow liquid were as follows.
¹H-NMR (CDCl₃) δ: 8.28 (d, 1H), 7.05-7.73 (m, 13H), 6.58-6.72 (m, 2H), 5.70 (dd, 2H), 5.21 (dd, 2H), 4.20 (d, 2H), 4.09 (s, 2H) .

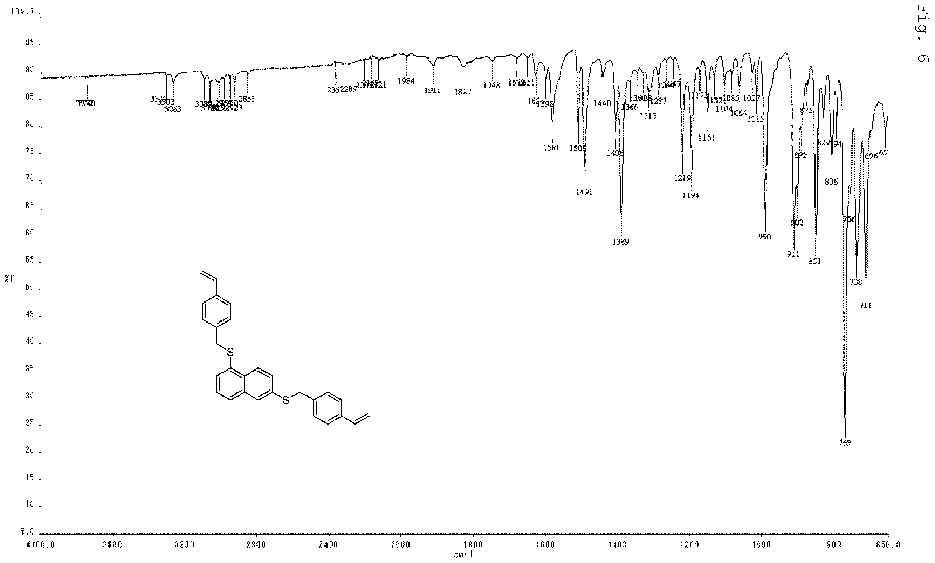

The IR spectral data of the pale yellow liquid were as shown in the chart of Fig. 6.

From these spectral data, the obtained pale yellow liquid was identified as being the title naphthalene compound represented by chemical formula (I-3-1).

### Example 7

### Synthesis of 1,6-bis(2-propyn-1-ylthio)naphthalene

961 mg (5.0 mmol) of 1,6-naphthalenedithiol, 1.19 g (10.0 mmol) of propargyl bromide, and 30 mL of dichloromethane were placed in a 100 mL flask, and 2.43 g (24.0 mmol) of triethylamine was added thereto while stirring. The mixture was then stirred at room temperature for 12 hours.

Subsequently, insoluble matter was filtered off at room temperature. The obtained filtrate was washed three times with 50 mL of water and concentrated under reduced pressure. The obtained concentrate was recrystallized with 20 mL of toluene to thus obtain 1.23 g of pale yellow crystals (yield: 91%).

The ¹H-NMR spectral data of the obtained pale yellow crystals were as follows.
¹H-NMR (CDCl₃) δ: 8.36 (d, 1H), 7.88 (d, 1H), 7.69-7.75 (m, 2H), 7.57 (dd, 1H), 7.44 (dd, 1H), 3.71 (d, 2H), 3.65 (d, 2H), 2.24 (t, 1H), 2.20 (t, 1H).

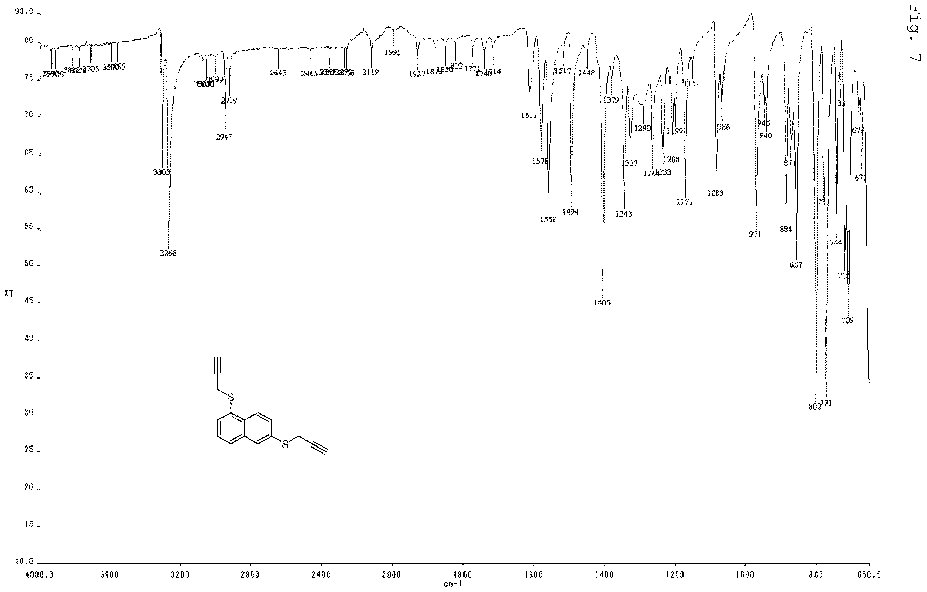

The IR spectral data of the pale yellow crystals were as shown in the chart of Fig. 7.

From these spectral data, the obtained pale yellow crystals were identified as being the title naphthalene compound represented by chemical formula (I-3-10).

### Example 8

### Synthesis of 1,6-bis(ethenylthio)naphthalene

961 mg (5.0 mmol) of 1,6-naphthalenedithiol, 19.79 g (200.0 mmol) of 1,2-dichloroethane, and 30 mL of dimethylformamide were placed in a 100 mL flask, and 1.42 g (14.0 mmol) of triethylamine was added thereto while stirring. The mixture was then heated to 60°C and stirred at the same temperature for 2 hours.

Thereafter, the mixture was cooled to room temperature, and 30 mL of toluene was added thereto. The mixture was then washed five times with 50 mL of water and concentrated under reduced pressure to thus obtain 1.32 g of a pale yellow liquid.

Thereafter, 1.32 g of the obtained pale yellow liquid and 20.8 mL of dimethylacetamide were placed in a 100 mL flask. The mixture was cooled to -10°C while stirring, and 1.31 g (11.7 mmol) of potassium t-butoxide was added in divided portions so as not to exceed 0°C. The mixture was then stirred at 0°C for 2 hours.

Thereafter, 21.8 mL of toluene was added, and the mixture was washed five times with 32.7 mL of water and concentrated under reduced pressure to thus obtain 982 mg of a pale yellow liquid (yield: 80%).

The ¹H-NMR spectral data of the obtained pale yellow liquid were as follows.
¹H-NMR (CDCl₃) δ: 8.17 (d, 1H), 8.05 (d, 1H), 7.95 (d, 1H), 7.67 (d, 1H), 7.54-7.60 (m, 2H), 6.87 (dd, 1H), 6.71 (dd, 1H), 5.38-5.56 (m, 3H), 5.11 (d, 1H).

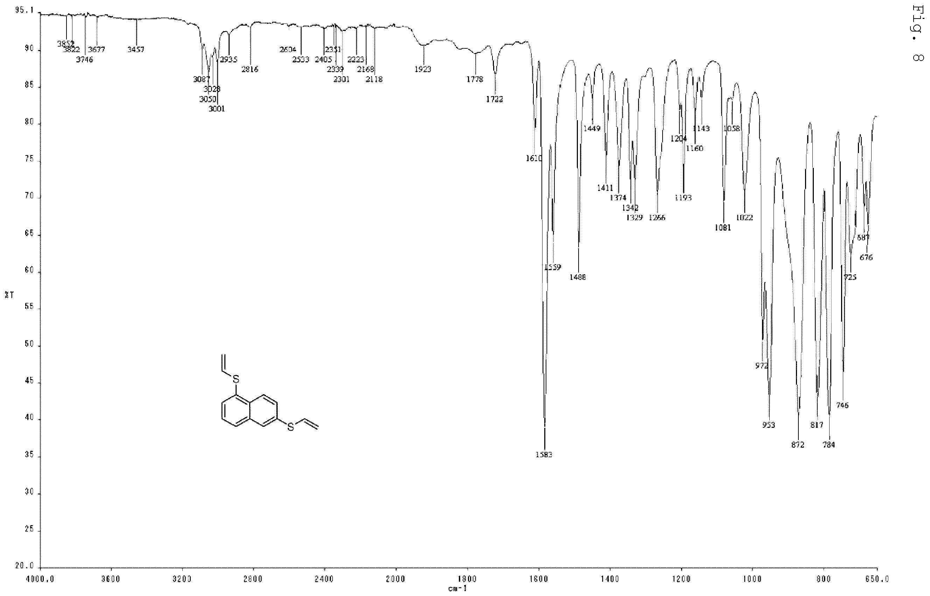

The IR spectral data of the pale yellow liquid were as shown in the chart of Fig. 8.

From these spectral data, the obtained pale yellow liquid was identified as being the title naphthalene compound represented by chemical formula (I-3-12).

### Example 9

### Synthesis of a mixture of the compounds represented by chemical formula (I-3-1), chemical formula (I-3-2), chemical formula (I-3-4), and chemical formula (I-3-7)

61.05 g (400.0 mmol) of CSM-P and 300 mL of dimethylformamide were placed in a 1000 mL flask, and 44.52 g (440.0 mmol) of triethylamine was added thereto while stirring. The mixture was then cooled to -10°C, and a solution of a mixture of 38.46 g (200.0 mmol) of 1,6-naphthalenedithiol and 100.00 g of dimethylformamide was added thereto dropwise so as not to exceed 0°C. The mixture was then stirred at 25°C for 12 hours.

Thereafter, the mixture was cooled to 15°C, and 4.17 g (40.0 mmol) of 35% hydrochloric acid was added at 20°C or lower. Insoluble matter was filtered off at room temperature, and the obtained filtrate was diluted with 600 mL of toluene, washed five times with 300 mL of water, and concentrated under reduced pressure to thus obtain 74.05 g of white crystals (yield: 87%).

The ¹H-NMR spectral data of the obtained white crystals were as follows.
¹H-NMR (CDCl₃) δ: 8.28 (d, 1H), 7.05-7.73 (m, 13H), 6.60-6.71 (m, 2H), 5.71 (dd, 1H), 5.67 (dd, 1H), 5.22 (d, 1H), 5.21 (dd, 1H), 4.21 (d, 2H), 4.10 (s, 2H).

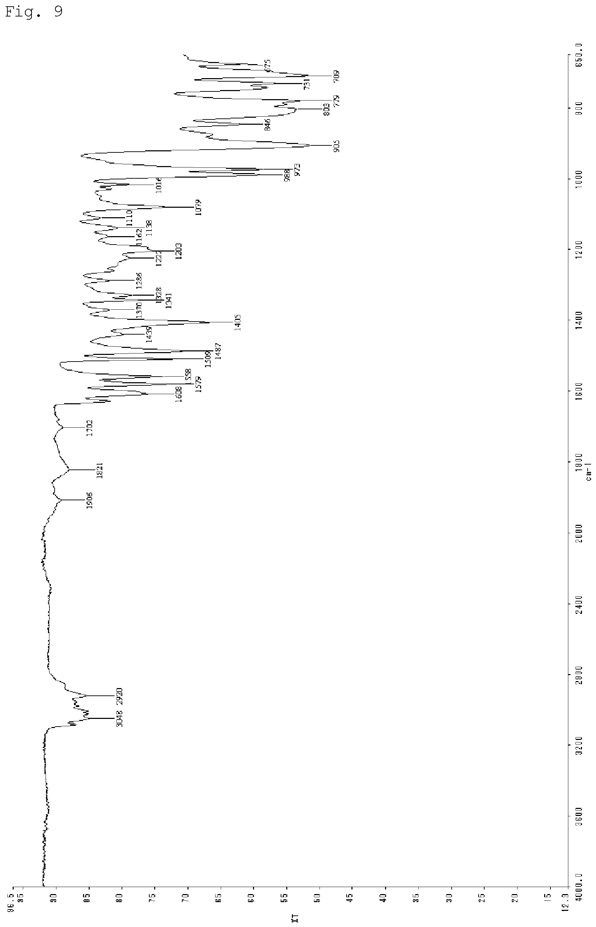

The IR spectral data of the white crystals were as shown in the chart of Fig. 9.

From these spectral data, the obtained white crystals were identified as being a mixture of the compound represented by chemical formula (I-3-1), the compound represented by chemical formula (I-3-2), the compound represented by chemical formula (I-3-4), and the compound represented by chemical formula (I-3-7).

### Examples 10 to 18

The naphthalene compounds synthesized in Examples 1 to 9 were subjected to evaluation testing (measurement of refractive index). The obtained test results were as shown in Table 3.

**Table 3**

| | | | Refractive index (25°C) |
|---|---|---|---|
| Example | 10 | The naphthalene compound of Example 1 | 1.72 |
| | 11 | The naphthalene compound of Example 2 | 1.63 |
| | 12 | The naphthalene compound of Example 3 | 1.68 |
| | 13 | The naphthalene compound of Example 4 | 1.76 |
| | 14 | The naphthalene compound of Example 5 | 1.65 |
| | 15 | The naphthalene compound of Example 6 | 1.70 |
| | 16 | The naphthalene compound of Example 7 | 1.65 |
| | 17 | The naphthalene compound of Example 8 | 1.70 |
| | 18 | The naphthalene compound of Example 9 | 1.71 |

The results shown in Table 3 indicate that the naphthalene compounds of Examples 1 to 9 all showed a high refractive index.

### Industrial Applicability

The naphthalene compound of the present invention is novel and is characterized by having a high refractive index. Polymerization of the composition containing the naphthalene compound can provide a cured product that is excellent in terms of expansion properties, heat resistance, and mechanical strength, and that has a high refractive index.

Thus, the composition of the present invention is suitable as a material for use in the production of coating materials, ink, adhesives, tackifiers, gas barrier films, color filters, optical films, optical lenses, touch panels, and the like.

## Claims

1. A naphthalene compound represented by chemical formula (I) : 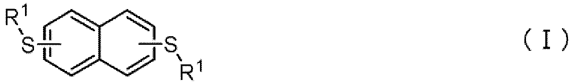 wherein R¹s (i) are the same or different and are each a group represented by formula (1), (ii) are the same and are each a group represented by formula (2), or (iii) are the same and are each a group represented by formula (3): 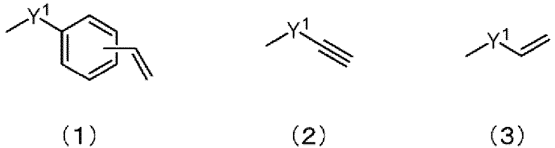 wherein Y¹s are the same and are each a single bond or a C₁-C₁₀ alkylene group.

2. A method for synthesizing the naphthalene compound of claim 1, comprising reacting a naphthalene dithiol compound represented by chemical formula (II) with a styrene compound represented by chemical formula (III), an alkyne compound represented by chemical formula (IV), or an alkene compound represented by chemical formula (V): 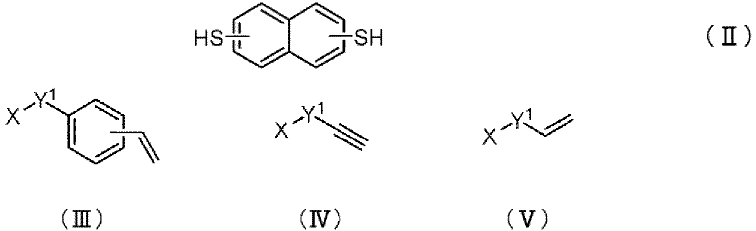 wherein Y¹ is as defined above, and X is a fluorine atom, a chlorine atom, a bromine atom, or an iodine atom.

3. A method for synthesizing the naphthalene compound of claim 1, the naphthalene compound being represented by chemical formula (I) wherein R¹s are the same and are each a group represented by formula (3),
the method comprising
reacting a naphthalene dithiol compound represented by chemical formula (II) with a C₂-C₁₂ dihaloalkane compound to haloalkylate two thiol groups (first step), and
reacting the resulting compound with a base (second step).

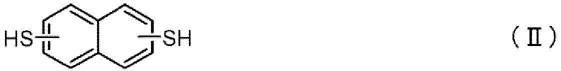

4. A composition containing the naphthalene compound of claim 1.
